# EUROPEAN PATENT SPECIFICATION

(11) **EP 2 100 902 B1**
(45) Date of publication and mention of the grant of the patent: **18.12.2019**
(21) Application number: 09163527.6
(22) Date of filing: 08.10.2003
(51) Int. Cl.: A61P 25/04, C07K 16/22, A61K 31/04, A61K 39/395, A61K 31/485

(54) **Methods for treating post-surgical pain by administering an antagonist antibody against the nerve growth factor and an opioid analgesic, and compositions containing the same**
Verfahren zur postoperativen Schmerzbehandlung durch Verabreichung eines antagonistischen Antikörpers gegen den Nervenwachstumsfaktor und ein Opioid-Analgetika, und diese enthaltende Zubereitungen
Méthode pour le traitement de la douleur post-chirurgical par administration d'un anticorps antagoniste du facteur de croissance neuronal et d'un analgésique opioide, et compositions les contenant

(30) Priority: 08.10.2002 US 417347 P
(43) Date of publication of application: 16.09.2009
(62) Divisional of application: 03816571.8
(73) Proprietor: Rinat Neuroscience Corp., South San Francisco, CA 94080 (US)
(72) Inventor: Shelton, David L., Oakland, CA 94618 (US); Vergara, German J., Moraga, CA 94556 (US)
(74) Representative: Pfizer

(56) References cited:
- WO-A-00/37103
- WO-A-01/78698
- US-A- 6 022 875
- J. HONGO ET AL.: "Antibody binding regions on human nerve growth factor identified by homolog- and alanine-scanning mutagenesis." HYBRIDOMA, vol. 19, no. 3, 2000, pages 215-227, XP002951932
- BRENNAN T J ET AL: "Role of nerve growth factor in a rat model for postoperative pain" SOCIETY FOR NEUROSCIENCE ABSTRACTS, vol. 24, no. 1-2, 1998, page 880, XP009071059 & 28TH ANNUAL MEETING OF THE SOCIETY FOR NEUROSCIENCE, PART 1; LOS ANGELES, CALIFORNIA, USA; NOVEMBER 7-12, 1998 ISSN: 0190-5295
- T. BRENNAN ET AL.: "Characterization of a rat model of incisional pain.", PAIN, vol. 64, no. 3, 1996, pages 493-501,
- G. TANDA ET AL.: "Combinations of cocaine with other dopamine uptake inhibitors: Assessment of additivity.", JOURNAL OF PHARMACOLOGY AND EXPERIMENTAL THERAPEUTICS, vol. 330, 2009, pages 802-809,
- G. SNEDECOR & W. COCHRAN: "Statistical Methods (sixth edition)", 1968, THE IOWA STATE UNIVERSITY PRESS, AMES, IOWA, USA * page 96 * * page 99 *
- T. BRENNAN: "Postoperative models of nociception", ILAR JOURNAL, vol. 40, no. 3, 1999, pages 129-136,

## Description

### FIELD OF THE INVENTION

This invention relates to methods and compositions for preventing or treating pain in a patient by administering a combination of a nerve growth factor antagonist and an opioid analgesic.

### BACKGROUND OF THE INVENTION

Severe pain is commonly treated with opioid analgesics which unfortunately have many undesirable side effects including reduced gastric motility, renal colic, mental clouding, and respiratory depression. Nerve growth factor (NGF) was the first neurotrophin identified, and its role in the development and survival of both peripheral and central neurons has been well characterized. NGF has been shown to be a critical survival and maintenance factor in the development of peripheral sympathetic and embryonic sensory neurons and of basal forebrain cholinergic neurons (Smeyne, et al., Nature 368:246-249 (1994); Crowley, et al., Cell 76:1001-1011 (1994)). NGF upregulates expression of neuropeptides in sensory neurons (Lindsay, et al., Nature 337:362-364 (1989)) and its activity is mediated through two different membrane-bound receptors, the TrkA tyrosine kinase receptor and the p75 receptor, which is structurally related to other members of the tumor necrosis factor receptor family (Chao, et al., Science 232:518-521 (1986)).

In addition to its effects in the nervous system, NGF has been increasingly implicated in processes outside of the nervous system. For example, exogenously administered NGF has been shown to enhance vascular permeability (Otten, et al., Eur. J. Pharmacol. 106:199-201 (1984)), enhance T- and B-cell immune responses (Otten, et al., Proc. Natl. Acad. Sci. U.S.A. 86:10059-10063 (1989)), induce lymphocyte differentiation and mast cell proliferation and cause the release of soluble biological signals from mast cells (Matsuda, et al., Proc. Natl. Acad. Sci. U.S.A. 85:6508-6512 (1988); Pearce, et al., J.Physiol. 372:379-393 (1986); Bischoff, et al., Blood 79:2662-2669 (1992); Horigome, et al., J. Biol. Chem. 268:14881-14887 (1993)).

NGF is produced by a number of cell types including mast cells (Leon, et al., Proc. Natl. Acad. Sci. U.S.A. 91:3739-3743 (1994)), B-lymphocytes (Torcia, et al., Cell 85:345-356 (1996), keratinocytes (Di Marco, et al., J. Biol. Chem. 268:22838-22846)), smooth muscle cells (Ueyama, et al., J. Hypertens. 11:1061-1065 (1993)), fibroblasts (Lindholm, et al., Eur. J. Neurosci. 2:795-801 (1990)), bronchial epithelial cells (Kassel, et al., Clin, Exp. Allergy 31:1432-40 (2001)), renal mesangial cells (Steiner, et al., Am. J. Physiol. 261:F792-798 (1991)) and skeletal muscle myotubes (Schwartz, et al., J Photochem. Photobiol. B 66:195-200 (2002)). NGF receptors have been found on a variety of cell types outside of the nervous system. For example, TrkA has been found on human monocytes, T- and B-lymphocytes and mast cells.

An association between increased NGF levels and a variety of inflammatory conditions has been observed in human patients as well as in several animal models. These include systemic lupus erythematosus (Bracci-Laudiero, et al., Neuroreport 4:563-565 (1993)), multiple sclerosis (Bracci-Laudiero, et al., Neurosci. Lett. 147:9-12 (1992)), psoriasis (Raychaudhuri, et al., Acta Derm. l'enereol. 78:84-86 (1998)), arthritis (Falcimi, et al., Ann. Rheum. Dis. 55:745-748 (1996)), interstitial cystitis (Okragly, et al., J. Urology 161:438-441 (1999)) and asthma (Braun, et al., Eur. J Immunol. 28:3240-3251 (1998)).

Consistently, an elevated level of NGF in peripheral tissues is associated with hyperalgesia and inflammation and has been observed in a number of forms of arthritis. The synovium of patients affected by rheumatoid arthritis expresses high levels of NGF while in non-inflamed synovium NGF has been reported to be undetectable (Aloe, et al., Arch. Rheum. 35:351-355 (1992)). Similar results were seen in rats with experimentally induced rheumatoid arthritis (Aloe, et al., Clin. Exp. Rheumatol. 10:203-204 (1992)). Elevated levels of NGF have been reported in transgenic arthritic mice along with an increase in the number of mast cells. (Aloe, el al., Int. J. Tissue Reactions-Exp. Clin. Aspects 15:139-143 (1993)).

There are two general categories of medication for the treatment of pain, each acting via different mechanisms and having differing effects, and both having disadvantages. The first category includes the nonsteroidal anti-inflammatory drugs (NSAIDs) which are used to treat mild pain, but whose therapeutic use is limited by undesirable gastrointestinal effects such as gastric erosion, formation of peptic ulcer or inflammation of the duodenum and of the colon and renal toxicity with prolonged use. The second category includes morphine and related opioids which are used to treat moderate to severe pain but whose therapeutic use is limited because of undesirable effects such as constipation, sedation, confusion, respiratory depression, mental clouding, renal colic, tolerance to prolonged use and risk of addiction. Compounds useful for treating pain with fewer or no side effects are therefore needed.

The opioid analgesics are a well-established class of analgesic agent. They are also sometimes referred to as opiates. The term opioid (interchangeably termed "opioid analgesic") is generally accepted to refer in a generic sense to all drugs, natural or synthetic, with morphine-like actions. The synthetic and semi-synthetic opioid analgesics are derivatives of five chemical classes of compound: phenanthrenes; phenylheptylamines; phenylpiperidines; morphinans; and benzomorphans. Pharmacologically these compounds have diverse activities, thus some are strong agonists at the opioid receptors (e.g. morphine); others are moderate to mild agonists (e.g. codeine); still others exhibit mixed agonist-antagonist activity (e.g. nalbuphine); and yet others are partial agonists (e.g. nalorphine). An opioid partial agonist such as nalorphine, (the N-alkyl analogue of morphine) may antagonize the analgesic effects of morphine, when given alone it can be a potent analgesic in its own right.

Of all of the opioid analgesics, morphine is the most widely used. Unfortunately, apart from its useful therapeutic properties, morphine also has a number of side effects. Another unfortunate characteristic is the development of tolerance and physical dependence that may limit the clinical use of such compounds. There is therefore a need to develop treatment methods using lower doses of opioid analgesics such as morphine, thereby reducing the incidence of side effects and the likelihood of tolerance and dependence, and thus avoiding the major problem of drug withdrawal associated with ceasing administration. There is a great need to develop other forms of enhancement of opioid pain treatment.

WO0037103 discloses a compound for delivery of therapeutic moieties, such as an opioid analgesic to nerve cells, for example to relieve pain. US6022875 discloses substituted 2,4-imidazolidinedione compounds as analgesics. Brennan et al (Society for Neuroscience Abstracts, 24(1-2), 1998, p880) discloses the effect of trkAIgG fusion protein in a rat model of incisional pain. Brennan et al (Pain, 64 (1996) 493-501) discloses the development and characterization of a rat model of incisional pain. Brennan (ILAR J 1999, 40(3), 129-136) further discusses this rat model of incisional pain and discloses that mechanical hyperalgesia is an important property of incisional pain.

### BRIEF SUMMARY OF THE INVENTION

The subject-matter of the invention is defined by the appended claims. The present invention is based upon the discovery that antagonists of NGF are effective in treating pain in conjunction with an opioid analgesic. Such therapy generally allows a reduced dosage of opioid analgesic to effect the same amount of pain reduction and/or other forms of enhancement of opioid pain treatment.

In a first aspect, the present invention features a nerve growth factor (NGF) antagonist and an opioid analgesic for use in treating post-surgical pain whereby the NGF antagonist and the opioid analgesic in combination provide effective pain relief; and wherein the NGF antagonist is an anti-NGF antibody which inhibits the binding of NGF to its trkA and p75 receptor *in vivo.* The relative amounts and ratios of NGF antagonist and opioid analgesic may vary. In some embodiments, enough NGF antagonist will be administered so as to allow reduction of the normal dose of opioid analgesic required to effect the same degree of pain amelioration by at least about 50%. In some embodiments, enough NGF antagonist will be administered so as to allow reduction of the normal dose of opioid analgesic required to effect the same degree of pain amelioration by at least about 5%, at least about 10%, at least about 20%, at least about 30%, at least about 40%, at least about 60%, at least about 70%, at least about 80%, or at least about 90%, or more.

In another aspect, the disclosure provides a nerve growth factor (NGF) antagonist and an opioid analgesic for use in enhancing opioid analgesic pain treatment comprising administering an effective amount of an opioid analgesic in conjunction with an effective amount of an anti-NGF antibody. Administration in conjunction, as used herein, comprises simultaneous administration and/or administration at different times. Administration in conjunction also encompasses administration as a co-formulation (i.e., the NGF antagonist and opioid analgesic are present (combined) in the same composition) and/or administration as separate compositions. As used herein, "administration in conjunction" is meant to encompass any circumstance wherein an opioid analgesic and NGF antagonist are administered in an effective amount to an individual. As further discussed herein, it is understood that the NGF antagonist and opioid analgesic can be administered at different dosing frequencies and/or intervals. For example, an anti-NGF antibody can be administered weekly, while an opioid analgesic can be administered more frequently. It is understood that the NGF antagonist and the opioid analgesic can be administered using the same route of administration or different routes of administration, and that different dosing regimens may change over the course of administration(s). Administration may be before the onset of pain.

In another aspect, the disclosure provides a nerve growth factor (NGF) antagonist and an opioid analgesic for use in reducing incidence of pain, ameliorating pain, palliating pain; and/or delaying the development or progression of pain in an individual, comprising administering an effective amount of an opioid analgesic in conjunction with an effective amount of an NGF antagonist, wherein the NGF antagonist is an anti-NGF antibody.

The medical uses of the disclosure are suitable for treating or preventing any pain of any etiology, including pain where the use of an opioid analgesic is generally prescribed, for example, pancreatitis, kidney stone, headache, dismennorhea, musculoskeletal pain (e.g., lower back pain), sprain, visceral pain, ovarian cysts, prostatitis, cystitis, chemical or thermal burns, or cancer (such as prostate cancer metastasized to bone, breast cancer that has metastasized to bone, lung cancer that has metastasized to bone, pancreatic cancer).

In an embodiment, the anti-NGF antibody recognizes human NGF. In yet other embodiments, the anti-NGF antibody specifically binds human NGF. In still further embodiments, the antibody binds essentially the same NGF epitope 6 as an antibody selected from any one or more of the following mouse monoclonal antibodies: MAb 911, MAb 912 and MAb 938 (*See* Hongo, et al., Hybridoma 19:215-227 (2000)). In some embodiment, the NGF antagonist binds NGF (such as hNGF) and does not significantly bind to related neurotrophins, such as NT-3, NT4/5, and/or BDNF. In still other embodiments, the anti-NGF antibody is humanized (such as antibody E3 described herein). In still other embodiments, the anti-NGF antibody is antibody E3 (as described herein). In other embodiments, the anti-NGF antibody comprises one or more CDR(s) of antibody E3 (such as one, two, three, four, five, or, in some embodiments, all six CDRs from E3). In other embodiments, the antibody is human. In still other embodiments, the anti-NGF antibody comprises the amino acid sequence of the heavy chain variable region shown in Table 1 (SEQ ID NO:1) and the amino acid sequence of the light chain variable region shown in Table 2 (SEQ ID NO:2). In still other embodiments, the antibody comprises a modified constant region, such as a constant region that is immunologically inert, e.g., does not trigger complement mediated lysis, or does not stimulate antibody-dependent cell mediated cytotoxicity (ADCC). In other embodiments, the constant region is modified as described in Eur. J. Immunol. (1999) 29:2613-2624; PCT Publication No. WO9958572.

In some embodiments, the NGF antagonist binds to the NGF molecule. In still other embodiments, the NGF antagonist is an antibody that binds specifically to NGF (such as human NGF). However, the NGF antagonist may alternatively bind to the trkA receptor. The NGF antagonist may be an anti-human NGF (anti-hNGF) monoclonal antibody that is capable of binding hNGF and effectively inhibiting the binding of hNGF to human TrkA (hTrkA) and/or effectively inhibiting activation of TrkA receptor.

The binding affinity of an anti-NGF antibody to NGF (such as hNGF) can be about 0.10 nM to about 0.80 nM, about 0.15 to about 0.75 nM and about 0.18 to about 0.72 nM. In one embodiment, the binding affinity is between about 2 pM and 22 pM. In some embodiment, the binding affinity is about 10 nM. In other embodiments, the binding affinity is less than about 10 nM. In other embodiments, the binding affinity is about 0.1 nM or about 0.07 nM. In other embodiments, the binding affinity is less than about 0.1 nM, or less than about 0.07 nM. In other embodiments, the binding affinity is any of about 100 nM, about 50 nM, about 10 nM, about 1 nM, about 500 pM, about 100 pM, or about 50 pM to any of about 2 pM, about 5 pM, about 10 pM, about 15 pM, about 20 pM, or about 40 pM. In some embodiments, the binding affinity is any of about 100 nM, about 50 nM, about 10 nM, about 1 nM, about 500 pM, about 100 pM, or about 50 pM, or less than about 50 pM. In some embodiments, the binding affinity is less than any of about 100 nM, about 50 nM, about 10 nM, about 1 nM, about 500 pM, about 100 pM, or about 50 pM. In still other embodiments, the binding affinity is about 2 pM, about 5 pM, about 10 pM, about 15 pM, about 20 pM, about 40 pM, or greater than about 40 pM. As is well known in the art, binding affinity can be expressed as K_{D}, or dissociation constant, and an increased binding affinity corresponds to a decreased K_{D}. The binding affinity of anti-NGF mouse monoclonal antibody 911 (Hongo et al., Hybridoma 19:215-227 (2000)) to human NGF is about 10 nM, and the binding affinity of humanized anti-NGF antibody E3 (described herein) to human NGF is about 0.07 nM.

The anti-NGF antibody may be an antibody fragment, including an antibody fragment selected from the group consisting of Fab, Fab', F(ab')2, Fv fragments, diabodies, single chain antibody molecules and multispecific antibodies formed from antibody fragments, and a single-chain Fv (scFv) molecule.

The opioid analgesic may be any compound exhibiting morphine-like biological activity. In some embodiments, the opioid analgesic is one or more of the following: morphine, codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine; or a pharmaceutically acceptable salt thereof.

The NGF antagonist and/or opioid analgesic can be administered to an individual via any suitable route. For example, they can be administered together or separately, and/or simultaneously and/or sequentially, orally, intravenously, sublingually, subcutaneously, intraarterially, intramuscularly, rectally, intraspinally, intrathoracically, intraperitoneally, intraventricularly, sublingually, transdermally or by inhalation. Administration can be systemic, e.g., intravenous, or localized.

In a second aspect, the present invention features compositions comprising an anti-NGF antibody and an opioid analgesic for use in treating post-surgical pain. The anti-NGF antibody and the opioid analgesic may be present together with one or more pharmaceutically acceptable carriers or excipients, or they may be present in separate compositions. In another aspect, the invention provides a synergistic composition of an anti-NGF antibody and an opioid analgesic.

In a third aspect, the present invention features a kit for use treating post-surgical pain, said kit comprising an anti-NGF antibody and an opioid analgesic. The kit may further comprise instructions for any of the methods described herein. The instructions may comprise administration of an anti-NGF antibody in conjunction with opioid (i.e., simultaneous administration and/or administration at different times). In some embodiments, the anti-NGF antibody and opioid analgesic are packaged together, but they may or may not be in the same container. Thus, in some embodiments, the kit comprises an anti-NGF antibody and an opioid analgesic present in the same container, and instructions for use in any of the methods described herein. In other embodiments, the kit comprises an anti-NGF antibody and an opioid present in separate containers.

### BRIEF DESCRIPTION OF THE DRAWINGS

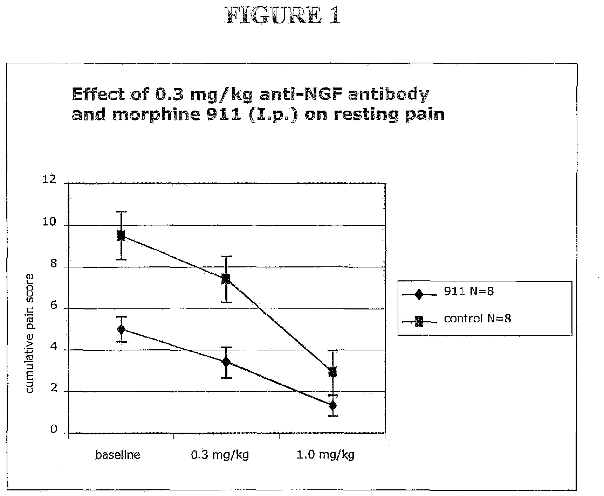

Figure 1 depicts that the cumulative pain score was reduced in animals treated with morphine in combination with an NGF antagonist, mouse anti-NGF antibody 911. Hongo, et al., Hybridoma 19:215-227 (2000). Pre-operative treatment with anti-NGF antibody ("911") alone at 0.3 mg/kg was more effective in reducing resting pain than 0.3 mg/kg morphine alone, and treatment with anti-NGF antibody in combination with morphine was more effective in reducing resting pain than morphine alone or anti-NGF antibody alone.

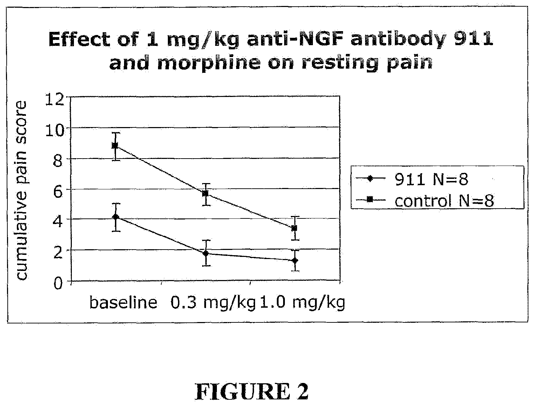

Figure 2 depicts that the cumulative pain score was reduced in animals treated with morphine in combination with an NGF antagonist, anti-NGF antibody 911. Treatment with anti-NGF antibody plus morphine was more effective in reducing resting pain than morphine alone or anti-NGF antibody alone.

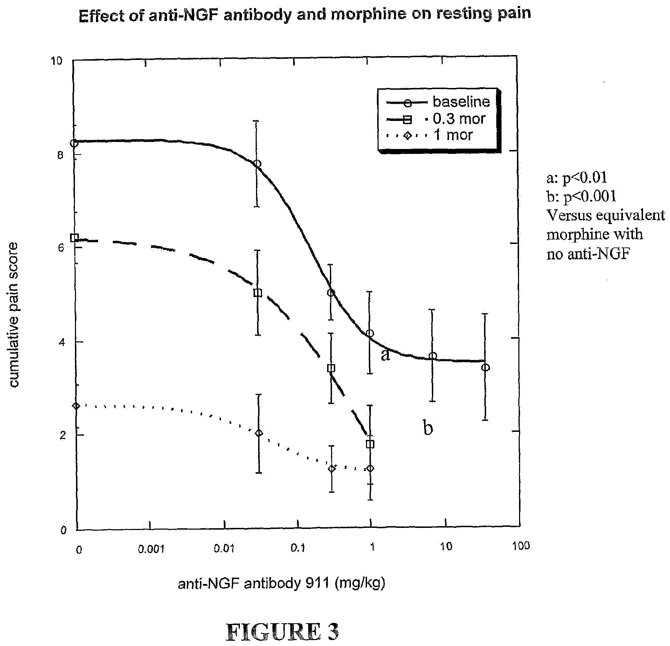

Figure 3 depicts the resting pain score observed following surgery and treatment with anti-NGF antibody and/or morphine. Animals were treated with an NGF antagonist, anti-NGF antibody 911, 15 hours prior to surgery and tested for pain behavior one day post-surgery. Animals were tested with no morphine (solid curve), 0.3 mg/kg morphine (dashed curve) or 1.0 mg/kg morphine (dotted curve). Treatment with 0.3 mg/kg of morphine in combination with either 0.3 mg/kg anti-NGF antibody or 1 mg/kg anti-NGF antibody significantly improved pain relief (i.e., reduced resting pain) as compared with treatment with 0.3 mg/kg morphine alone. In addition, anti-NGF antibody treatment alone provided pain relief equivalent to morphine dosed at 0.3 mg/kg. Treatment with anti-NGF antibody at 1.0 mg/kg plus 0.3 mg/kg morphine yielded pain relief at least equal to that obtained with 1 mg/kg of morphine alone.

### DETAILED DESCRIPTION OF THE INVENTION

We have discovered that pain may be treated or prevented by administering an effective amount of an anti-NGF antibody in conjunction with an opioid analgesic. The medical uses and compositions of the present disclosure are useful for the treatment or prevention of pain where the use of an opioid analgesic is generally prescribed. By the use of an anti-NGF antibody and an opioid analgesic in conjunction, in accordance with the present disclosure, it is now possible to treat pain with a lower dose of an opioid analgesic thereby reducing the likelihood of side-effects associated with opioid analgesic usage (e.g. respiratory depression, constipation, renal colic, nausea and vomiting, and tolerance and dependence and the associated problem of drug withdrawal). In some embodiments, enough anti-NGF antibody will be administered so as to allow reduction of the normal dose of opioid analgesic required to effect the same degree of pain amelioration by at least about 5%, at least about 10%, at least about 20%, at least about 30%, at least about 50%, at least about 60%, at least about 70%, at least about 80%, or at least about 90%, or more.

The treatment of pain with an opioid analgesic can also be enhanced as described herein, by administration of the opioid analgesic in conjunction with an anti-NGF antibody.

The invention provides an nerve growth factor (NGF) antagonist and an opioid analgesic for use in treating post-surgical pain in an individual (including a mammal, both human and non-human), wherein the NGF antagonist is an anti-NGF antibody. The disclosure provides medical uses for enhancing opioid analgesic treatment or prevention of pain in an individual comprising administering an effective amount of an anti-NGF antibody in conjunction with an effective amount of an opioid analgesic. The disclosure provides methods of preventing, ameliorating and/or preventing the development or progression of pain.

In some embodiments, the anti-NGF antibody is capable of binding NGF and effectively inhibiting the binding of NGF to its TrkA and/or p75 receptor *in vivo*, and/or effectively inhibiting NGF from activating its TrkA and/or p75 receptor.

In another aspect, the disclosure provides medical uses for ameliorating, delaying the development of and/or preventing the progression of pain.

Exemplary opioid analgesics of the present invention include, but are not limited to, morphine, codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine; or a pharmaceutically acceptable salt thereof. In some embodiments, the opioid analgesic is morphine; or a pharmaceutically acceptable salt thereof.

Exemplary salts of opioid analgesics of use in the present invention include morphine sulphate, morphine hydrochloride, morphine tartrate, codeine phosphate, codeine sulphate, dihydrocodeine bitartrate, diacetylmorphine hydrochloride, hydrocodone bitartrate, hydromorphone hydrochloride, levorphanol tartrate, oxymorphone hydrochloride, alfentanil hydrochloride, buprenorphine hydrochloride, butorphanol tartrate, fentanyl citrate, meperidine hydrochloride, methadone hydrochloride, nalbuphine hydrochloride, propoxyphene hydrochloride, propoxyphene napsylate (2-naphthalenesulphonic acid (1:1) monohydrate), and pentazocine hydrochloride. In one embodiment, the opioid analgesic is morphine sulphate.

The medical uses and compositions of the present disclosure are useful for the treatment of pain of any etiology, including acute and chronic pain, any pain with an inflammatory component, and any pain in which an opioid analgesic is usually prescribed. Examples of pain include post-surgical pain, post-operative pain (including dental pain), migraine, headache and trigeminal neuralgia, pain associated with burn, wound or kidney stone, pain associated with trauma (including traumatic head injury), neuropathic pain, pain associated with musculo-skeletal disorders such as rheumatoid arthritis, osteoarthritis, cystitis, pancreatitis, inflammatory bowel disease, ankylosing spondylitis, sero-negative (non-rheumatoid) arthropathies, non-articular rheumatism and peri-articular disorders, and pain associated with cancer (including "break-through pain" and pain associated with terminal cancer), peripheral neuropathy and post-herpetic neuralgia. Examples of pain with an inflammatory component (in addition to some of those described above) include rheumatic pain, pain associated with mucositis, and dysmenorrhea. The medical uses and compositions of the present invention are used for treatment of post-surgical pain.

The anti-NGF antibody and/or opioid analgesic can be administered to an individual via any suitable route. For example, they can be administered together or separately, and/or simultaneously and/or sequentially, orally, intravenously, sublingually, subcutaneously, intraarterially, intramuscularly, rectally, intraspinally, intrathoracically, intraperitoneally, intraventricularly, sublingually, transdermally or by inhalation. Administration can be systemic, e.g., intravenous, or localized.

In another aspect, the invention provides compositions and kits for use in treating post-surgical pain comprising an anti-NGF antibody and an opioid analgesic. In some embodiments, the anti-NGF antibody is capable of effectively inhibiting NGF binding to its TrkA and/or p75 receptor(s) and/or of effectively inhibiting NGF from activating its TrkA and/or p75 receptor(s).

### General Techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press*;* Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J.D. Capra, eds., Harwood Academic Publishers, 1995).

### Definitions

An "antibody" (interchangeably used in plural form) is an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact polyclonal or monoclonal antibodies, but also fragments thereof (such as Fab, Fab', F(ab')2, Fv), single chain (ScFv), mutants thereof, fusion proteins comprising an antibody portion, humanized antibodies, chimeric antibodies, diabodies linear antibodies, single chain antibodies, multispecific antibodies (e.g., bispecific antibodies) and any other modified configuration of the immunoglobulin molecule that comprises an antigen recognition site of the required specificity. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these maybe further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

A "monoclonal antibody" refers to a homogeneous antibody population wherein the monoclonal antibody is comprised of amino acids (naturally occurring and non-naturally occurring) that are involved in the selective binding of an antigen. A population of monoclonal antibodies is highly specific, being directed against a single antigenic site. The term "monoclonal antibody" encompasses not only intact monoclonal antibodies and full-length monoclonal antibodies, but also fragments thereof (such as Fab, Fab', F(ab')2, Fv), single chain (ScFv), mutants thereof, fusion proteins comprising an antibody portion, humanized monoclonal antibodies, chimeric monoclonal antibodies, and any other modified configuration of the immunoglobulin molecule that comprises an antigen recognition site of the required specificity and the ability to bind to an antigen. It is not intended to be limited as regards to the source of the antibody or the manner in which it is made (e.g., by hybridoma, phage selection, recombinant expression, transgenic animals, etc.).

"Humanized" antibodies refer to a molecule having an antigen binding site that is substantially derived from an immunoglobulin from a non-human species and the remaining immunoglobulin structure of the molecule based upon the structure and/or sequence of a human immunoglobulin. The antigen binding site may comprise either complete variable domains fused onto constant domains or only the complementarity determining regions (CDRs) grafted onto appropriate framework regions in the variable domains. Antigen binding sites may be wild type or modified by one or more amino acid substitutions, e.g., modified to resemble human immunoglobulin more closely. Some forms of humanized antibodies preserve all CDR sequences (for example, a humanized mouse antibody which contains all six CDRs from the mouse antibodies). Other forms of humanized antibodies have one or more CDRs (one, two, three, four, five, six) which are altered with respect to the original antibody. In some instances, framework region (FR) residues or other residues of the human immunoglobulin replaced by corresponding non-human residues. Furthermore, humanized antibodies may comprise residues which are not found in the recipient antibody or in the donor antibody.

As used herein, the term "nerve growth factor" and "NGF" refers to nerve growth factor and variants thereof that retain at least part of the activity of NGF. As used herein, NGF includes all mammalian species of native sequence NGF, including human, canine, feline, equine, or bovine.

"NGF receptor" refers to a polypeptide that is bound by or activated by NGF. NGF receptors include the TrkA receptor and the p75 receptor of any mammalian species, including, but are not limited to, human, canine, feline, equine, primate, or bovine.

An "NGF antagonist" refers to any molecule that blocks, suppresses or reduces (including significantly) NGF biological activity, including downstream pathways mediated by NGF signaling, such as receptor binding and/or elicitation of a cellular response to NGF. The term "antagonist" implies no specific mechanism of biological action whatsoever, and is deemed to expressly include and encompass all possible pharmacological, physiological, and biochemical interactions with NGF whether direct or indirect, or whether interacting with NGF, its receptor, or through another mechanism, and its consequences which can be achieved by a variety of different, and chemically divergent, compositions. Exemplary NGF antagonists include, but are not limited to, an anti-NGF antibody, an anti-sense molecule directed to an NGF (including an anti-sense molecule directed to a nucleic acid encoding NGF), an NGF inhibitory compound, an NGF structural analog, a dominant-negative mutation of a TrkA receptor that binds an NGF, a TrkA immunoadhesin, an anti-TrkA antibody, an anti-p75 antibody, and a kinase inhibitor. For purpose of the present disclosure, it will be explicitly understood that the term "antagonist" encompass all the previously identified terms, titles, and functional states and characteristics whereby the NGF itself, an NGF biological activity (including but not limited to its ability to mediate any aspect of pain), or the consequences of the biological activity, are substantially nullified, decreased, or neutralized in any meaningful degree. In some embodiments, an NGF antagonist i.e., an antibody) binds (physically interacts with) NGF, binds to an NGF receptor (such as trkA receptor and/or p75 receptor), reduces (impedes and/or blocks) downstream NGF receptor signaling, and/or inhibits (reduces) NGF synthesis, production or release. In other embodiments, an NGF antagonist binds NGF and prevents TrkA receptor dimerization and/or TrkA autophosphorylation. In other embodiments, an NGF antagonist inhibits or reduces NGF synthesis and/or production (release). Examples of types of NGF antagonists are provided herein.

As used herein, an "anti-NGF antibody" refers to an antibody which is able to bind to NGF and inhibit NGF biological activity and/or downstream pathway(s) mediated by NGF signaling.

A "TrkA immunoadhesin" refers to a soluble chimeric molecule comprising a fragment of a TrkA receptor, for example, the extracellular domain of a TrkA receptor and an immunoglobulin sequence, which retains the binding specificity of the TrkA receptor.

"Biological activity" of NGF generally refers to the ability to bind NGF receptors and/or activate NGF receptor signaling pathways. Without limitation, a biological activity includes any one or more of the following: the ability to bind an NGF receptor (such as p75 and/or TrkA); the ability to promote TrkA receptor dimerization and/or autophosphorylation; the ability to activate an NGF receptor signaling pathway; the ability to promote cell differentiation, proliferation, survival, growth and other changes in cell physiology, including (in the case of neurons, including peripheral and central neuron) change in neuronal morphology, synaptogenesis, synaptic function, neurotransmitter and/or neuropeptide release and regeneration following damage; and the ability to mediate pain.

The term "epitope" is used to refer to binding sites for (monoclonal or polyclonal) antibodies on protein antigens.

As used herein, "treatment" is an approach for obtaining beneficial or desired clinical results. For purposes of this disclosure, beneficial or desired clinical results include, but are not limited to, one or more of the following: improvement or alleviation of any aspect of pain, including acute, chronic, inflammatory, neuropathic, or post-surgical pain. For purposes of this invention, beneficial or desired clinical results include, but are not limited to, one or more of the following: lessening severity, alleviation of one or more symptoms associated with pain including any aspect of pain (such as shortening duration of pain, and/or reduction of pain sensitivity or sensation).

"Reducing incidence" of pain means any of reducing severity (which can include reducing need for and/or amount of (e.g., exposure to) other drugs and/or therapies generally used for this conditions), duration, and/or frequency (including, for example, delaying or increasing time to pain in an individual). As is understood by those skilled in the art, individuals may vary in terms of their response to treatment, and, as such, for example, a "method of reducing incidence of pain in an individual" reflects administering the NGF antagonist described herein in conjunction with an opioid analgesic as described herein, based on a reasonable expectation that such administration may likely cause such a reduction in incidence in that particular individual.

"Ameliorating" pain or one or more symptoms of pain means a lessening or improvement of one or more symptoms of a pain as compared to not administering an NGF antagonist in conjunction with an opioid analgesic. "Ameliorating" also includes shortening or reduction in duration of a symptom.

"Palliating" pain or one or more symptoms of pain means lessening the extent of one or more undesirable clinical manifestations of pain in an individual or population of individuals treated with an NGF antagonist in conjunction with an opioid analgesic in accordance with the invention.

As used therein, "delaying" the development of pain means to defer, hinder, slow, retard, stabilize, and/or postpone progression of pain. This delay can be of varying lengths of time, depending on the history of the disease and/or individuals being treated. As is evident to one skilled in the art, a sufficient or significant delay can, in effect, encompass prevention, in that the individual does not develop pain. A method that "delays" development of the symptom is a method that reduces probability of developing the symptom in a given time frame and/or reduces extent of the symptoms in a given time frame, when compared to not using the method. Such comparisons are typically based on clinical studies, using a statistically significant number of subjects.

"Development" or "progression" of pain means initial manifestations and/or ensuing progression of the disorder. Development of pain can be detectable and assessed using standard clinical techniques as well known in the art. However, development also refers to progression that may be undetectable. For purpose of this invention, development or progression refers to the biological course of the symptoms. "Development" includes occurrence, recurrence, and onset. As used herein "onset" or "occurrence" of pain includes initial onset and/or recurrence.

An "effective amount" is an amount sufficient to effect beneficial or desired clinical results including alleviation or reduction in the pain sensation. For purposes of this disclosure, an effective amount of an NGF antagonist and an opioid includes an amount sufficient to treat, ameliorate, reduce the intensity of, or prevent pain (including nociception and the sensation of pain) of any sort, including acute, chronic, inflammatory, neuropathic, or post-surgical pain. In some embodiments, an effective amount of an opioid analgesic and an NGF antagonist is a quantity of the NGF antagonist and the opioid analgesic capable of modulating the sensitivity threshold to external stimuli to a level comparable to that observed in healthy subjects. In other embodiments, this level may not be comparable to that observed in healthy subjects, but is reduced compared to not receiving the combination therapy. An effective amount of an NGF antagonist also encompasses an amount of an NGF antagonist sufficient to enhance opioid treatment (therapeutic effect) of pain, as described herein, or to reduce the dose of opioid necessary for treatment or prevention of pain, as described herein. As is understood in the art, an effective amount of NGF antagonist in conjunction with opioid may vary, depending on, inter alia, type of pain (and patient history as well as other factors such as the type (and/or dosage) or NGF antagonist and/or opioid used. An effective amount, in the context of this invention, may also be amounts of an NGF antagonist and an opioid antagonist such that synergy is achieved. An effective amount of an antagonist in the context of this invention generally means an amount sufficient to result in enhancement of a therapeutic effect of an opioid for pain (which can, in turn, mean that dosage is reduced and/or some other beneficial effect is observed) and/or result in a beneficial effect as compared to opioid treatment alone. An "effective amount" of an NGF antagonist can also result in a synergistic effect as compared to administering NGF antagonist or opioid alone. In some embodiments, an "effective amount" is an amount sufficient to delay development of pain.

An "individual" is a vertebrate, preferably a mammal, more preferably a human. Mammals include, but are not limited to, farm animals, sport animals, pets, primates, horses, cows, dogs, cats, mice and rats.

The term "opioid analgesic" refers to all drugs, natural or synthetic, with morphine-like actions. The synthetic and semi-synthetic opioid analgesics are derivatives of five chemical classes of compound: phenanthrenes; phenylheptylamines; phenylpiperidines; morphinans; and benzomorphans, all of which are within the scope of the term. Exemplary opioid analgesics include codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine or pharmaceutically acceptable salts thereof.

As used herein, administration "in conjunction" includes simultaneous administration and/or administration at different times. Administration in conjunction also encompasses administration as a co-formulation (i.e., the NGF antagonist and opioid are present in the same composition) or administration as separate compositions. As used herein, administration in conjunction is meant to encompass any circumstance wherein an opioid and NGF antagonist are administered to an individual, which can occur simultaneously and/or separately. As further discussed herein, it is understood that the NGF antagonist and opioid can be administered at different dosing frequencies or intervals. For example, an anti-NGF antibody can be administered weekly, while an opioid can be administered more frequently. It is understood that the NGF antagonist and the opioid analgesic can be administered using the same route of administration or different routes of administration.

"Post-surgical pain" (interchangeably termed "post-incisional pain") refers to pain arising or resulting from all surgical procedures, whether invasive or non-invasive. As used herein, post-surgical pain does not include pain that occurs (arises or originates) without an external physical trauma. In some embodiments, post-surgical pain is internal or external (including peripheral) pain, and the wound, cut, trauma, tear or incision may occur deliberately (as with a surgical incision). As used herein, "pain" includes nociception and the sensation of pain, and pain can be assessed objectively and subjectively, using pain scores and other methods well-known in the art. Post-surgical pain, as used herein, includes allodynia (i.e., increased response to a normally non-noxious stimulus) and hyperalgesia (i.e., increased response to a normally noxious or unpleasant stimulus), which can in turn, be thermal or mechanical (tactile) in nature. In some embodiments, the pain is characterized by thermal sensitivity, mechanical sensitivity and/or resting pain. In some embodiments, the post-surgical pain comprises mechanically-induced pain or resting pain. In other embodiments, the post-surgical pain comprises resting pain. The pain can be primary or secondary pain, as is well-known in the art.

Opioid treatment of pain is "enhanced" when an aspect of opioid treatment is improved (as compared to administering opioid without administering an NGF antagonist). For example, efficacy of opioid treatment of pain may be increased in the presence of NGF antagonist relative to efficacy of a opioid analgesic in the absence of NGF antagonist. As another example, treatment or prevention of pain with an opioid may be "enhanced" by the use of an NGF antagonist in conjunction with the opioid analgesic when that use permits better pain relief (for example, when a dose of opioid is used that does not permit effective treatment or prevention of pain).

### Medical uses of the Invention

With respect to all uses described herein, reference to an NGF antagonists and opioid analgesics also include compositions comprising one or more of these agents. The present invention is useful for treating post-surgical pain in individuals including all mammals, both human and non-human.

In one aspect, the invention provides an anti-NGF antibody and an opioid analgesic for use in treating post-surgical pain in an individual. In some embodiments, enough anti-NGF antibody will be administered so as to allow reduction of the normal dose of opioid analgesic required to effect the same degree of pain amelioration by at least about 5%, at least about 10%, at least about 20%, at least about 30%, at least about 50%, at least about 60%, at least about 70%, at least about 80%, or at least about 90%, or more.

In another aspect, the disclosure provides for enhancing opioid analgesic treatment of pain in an individual comprising administering an effective amount of an anti-NGF antibody in conjunction with an effective amount of an opioid analgesic.

In some aspects, pain comprises any one or more of the following: acute and/or chronic pain, any pain with an inflammatory component, post-surgical pain, post-operative pain (including dental pain), migraine, headache and trigeminal neuralgia, pain associated with burn, wound or kidney stone, pain associated with trauma (including traumatic head injury), neuropathic pain, and pain associated with cancer (including "break-through pain" and pain associated with terminal cancer). In other aspects, the pain is any pain that is usually treated with an opioid analgesic (such as morphine), for example, pancreatitis, kidney stone, headache, dismennorhea, musculoskeletal pain (e.g., lower back pain), sprain, visceral pain, ovarian cysts, prostatitis, cystitis, chemical or thermal burns, cancer (such as prostate cancer metastasized to bone, breast cancer that has metastasized to bone, lung cancer that has metastasized to bone, pancreatic cancer).

In another aspect, the disclosure provides for preventing, ameliorating and/or preventing the development or progression of pain. Thus, in some embodiments, the NGF antagonist, such as an anti-NGF antibody, and/or opioid analgesic are administered prior to a painful event (such as surgery). For example, the NGF antagonist can be administered 30 minutes, one hour, 5 hours, 10 hours, 15 hours, 24 hours or even more, such as 1 day, several days, or even a week, 2 weeks, 3 weeks, or more prior to the activity likely to result in, or at a risk of causing, pain (such as external trauma or an operation).

Treatment or prevention of pain is assessed using methods well-known in the art. Assessment may be performed based on objective measure, such as observation of behavior such as reaction to stimuli, facial expressions and the like. Assessment may also be based on subjective measures, such as patient characterization of pain using various pain scales. *See*, *e.g*., Katz et al, Surg Clin North Am. (1999) 79 (2):231-52; Caraceni et al. J Pain Symptom Manage (2002) 23(3):239-55.

It is understood that when an anti-NGF antibody and an opioid analgesic are administered in conjunction, either as a single or as separate composition(s), the nerve growth factor antagonist and the opioid analgesic are presented in a ratio which is consistent with the manifestation of the desired effect. In some embodiments, the ratio by weight of the nerve growth factor antagonist to the opioid analgesic may be approximately 1 to 1. In some embodiments, this ratio may be between about 0.001 to about 1 and about 1000 to about 1, or between about 0.01 to about 1 and about 100 to about 1. Other ratios are contemplated.

It will be appreciated that the amount of an anti-NGF antibody and opioid analgesic required for use in the treatment or prevention of pain will vary not only with the particular compounds or compositions selected but also with the route of administration, the nature of the condition being treated, and the age and condition of the patient, and will ultimately be at the discretion of the attendant physician.

### NGF antagonists

The present invention relates to anti-NGF antibodies as NGF antagonists. Other NGF antagonists refers to any molecule that blocks, suppresses or reduces (including significantly) NGF biological activity, including downstream pathways mediated by NGF signaling, such as receptor binding and/or elicitation of a cellular response to NGF are also described herein. The term "antagonist" implies no specific mechanism of biological action whatsoever, and is deemed to expressly include and encompass all possible pharmacological, physiological, and biochemical interactions with NGF and its consequences which can be achieved by a variety of different, and chemically divergent, compositions. Exemplary NGF antagonists include, but are not limited to, an anti-NGF antibody, an anti-sense molecule directed to NGF (including an anti-sense molecule directed to a nucleic acid encoding NGF), an anti-sense molecule directed to an NGF receptor (such as TrkA receptor and/or p75 receptor) (including an anti-sense molecule directed to a nucleic acid encoding TrkA and/or p75), an NGF inhibitory compound, an NGF structural analog, a dominant-negative mutation of a TrkA receptor that binds an NGF, a TrkA immunoadhesin, an anti-TrkA antibody, an anti-p75 antibody, and a kinase inhibitor. For purpose of the present disclosure, it will be explicitly understood that the term "antagonist" encompasses all the previously identified terms, titles, and functional states and characteristics whereby the NGF itself, an NGF biological activity (including but not limited to its ability to mediate any aspect of pain), or the consequences of the biological activity, are substantially nullified, decreased, or neutralized in any meaningful degree. In some embodiments, an NGF antagonist (i.e., an antibody) binds (physically interacts with) NGF, or in some aspects it binds to an NGF receptor (such as TrkA and/or p75 receptor), and/or reduces (impedes and/or blocks) downstream NGF receptor signaling. Accordingly, in some embodiments, an NGF antagonist binds (physically interacts with) NGF. In other aspect, an NGF antagonist binds to an NGF receptor (such as trkA receptor or p75). In other embodiments, an NGF antagonist reduces (impedes and/or blocks) downstream NGF receptor signaling (*e.g*., inhibitors of kinase signaling). In other aspects, an NGF antagonist inhibits (reduces) NGF synthesis and/or release. In another aspect, the NGF antagonist is a TrkA immunoadhesin. In another aspect, the NGF antagonist is other than an anti-NGF antibody. In some embodiment, the NGF antagonist binds NGF (such as hNGF) and does not significantly bind to related neurotrophins, such as NT-3, NT4/5, and/or BDNF. In other embodiments, the NGF antagonist is an anti-NGF antibody. In still other embodiments, the anti-NGF antibody is humanized (such as antibody E3 described herein). In some embodiments, the anti-NGF antibody is antibody E3 (as described herein). In other embodiments, the anti-NGF antibody comprises one or more CDR(s) of antibody E3 (such as one, two, three, four, five, or, in some embodiments, all six CDRs from E3). In other embodiments, the antibody is human. In still other embodiments, the anti-NGF antibody comprises the amino acid sequence of the heavy chain variable region shown in Table 1 (SEQ ID NO:1) and the amino acid sequence of the light chain variable region shown in Table 2 (SEQ ID NO:2). In still other embodiments, the antibody comprises a modified constant region, such as a constant region that is immunologically inert, e.g., does not trigger complement mediated lysis, or does not stimulate antibody-dependent cell mediated cytotoxicity (ADCC). In other embodiments, the constant region is modified as described in Eur. J. Immunol. (1999) 29:2613-2624; PCT Publication No. WO9958572.

### Anti-NGF antibodies

In the present invention, the NGF antagonist comprises an anti-NGF antibody. An anti-NGF antibody should exhibit any one or more of the following characteristics: (a) bind to NGF; (b) inhibit NGF biological activity or downstream pathways mediated by NGF signaling function; (c) treating or preventing any aspect of pain, particularly in conjunction with an opioid analgesic; (d) block or decrease NGF receptor activation (including TrkA receptor dimerization and/or autophosphorylation); (e) increase clearance of NGF; (f) inhibit (reduce) NGF synthesis, production or release; (g) enhance opioid treatment of pain.

Anti-NGF antibodies are known in the art, *see,* e.g., PCT Publication Nos. WO 01/78698, WO 01/64247, U.S. Patent No. 5,844,092, 5,877,016, and 6,153,189; Hongo et al., Hybridoma, 19:215-227 (2000); Cell. Molec. Biol. 13:559-568 (1993); GenBank Accession Nos. U39608, U39609, L17078, or L17077.

In some embodiments, the anti-NGF antibody is a humanized mouse anti-NGF monoclonal antibody termed antibody "E3", which comprises the human heavy chain IgG2a constant region containing the following mutations: A330P331 to S330S331 (amino acid numbering with reference to the wildtype IgG2a sequence; *see* Eur. J. Immunol. (1999) 29:2613-2624); the human light chain kappa constant region; and the heavy and light chain variable regions shown in Tables 1 and 2.

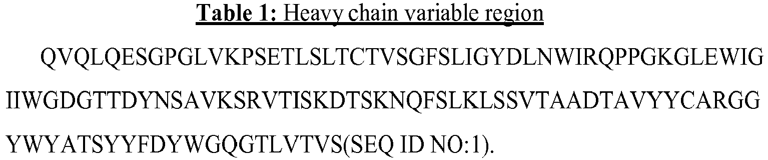

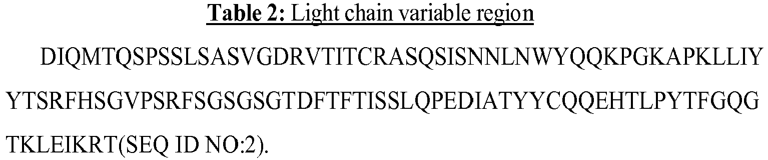

The following polynucleotides encoding the E3 heavy chain variable region or the E3 light chain variable region were deposited at the ATCC on January 8, 2003:

| ***Material*** | | ATCC Accession No. | Date of Deposit |
|---|---|---|---|
| Vector Eb.911.3E | E3 light chain V region | PTA-4893 | January 8, 2003 |
| Vector Eb.pur.911.3E | E3 light chain V region | PTA-4894 | January 8, 2003 |
| Vector Db.911.3E | E3 heavy chain V region | PTA-4895 | January 8, 2003 |

Vector Eb.911.3E is a polynucleotide encoding the light chain variable region shown in Table 2; vector Eb.pur.911.3E is a polynucleotide encoding the light chain variable region shown in Table 2 and vector Db.911.3E is a polynucleotide encoding the heavy chain variable region shown in Table 1.

In another embodiment, the anti-NGF antibody comprises one or more CDR(s) of antibody E3 (such as one, two, three, four, five, or, in some embodiments, all six CDRs from E3). Determination of CDR regions is well within the skill of the art.

The antibodies useful in the present invention can encompass monoclonal antibodies, polyclonal antibodies, antibody fragments (e.g., Fab, Fab', F(ab')2, Fv, Fc, etc.), chimeric antibodies, bispecific antibodies, heteroconjugate antibodies, single chain (ScFv), mutants thereof, fusion proteins comprising an antibody portion, humanized antibodies, and any other modified configuration of the immunoglobulin molecule that comprises an antigen recognition site of the required specificity, including glycosylation variants of antibodies, amino acid sequence variants of antibodies, and covalently modified antibodies. The antibodies may be murine, rat, human, or any other origin (including chimeric or humanized antibodies). For purposes of this invention, the antibody reacts with NGF in a manner that inhibits NGF and/or downstream pathways mediated by the NGF signaling function. In one embodiment, the antibody is a human antibody which recognizes one or more epitopes on human NGF. In another embodiment, the antibody is a mouse or rat antibody which recognizes one or more epitopes on human NGF. In another embodiment, the antibody recognizes one or more epitopes on an NGF selected from the group consisting of: primate, canine, feline, equine, and bovine. In other embodiments, the antibody comprises a modified constant region, such as a constant region that is immunologically inert, e.g., does not trigger complement mediated lysis, or does not stimulate antibody-dependent cell mediated cytotoxicity (ADCC). ADCC activity can be assessed using methods disclosed in U.S. Patent No. 5, 500, 362. In other embodiments, the constant region is modified as described in Eur. J. Immunol. (1999) 29:2613-2624; PCT Publication No. WO9958572.

The binding affinity of an anti-NGF antibody to NGF (such as hNGF) can be about 0.10 to about 0.80 nM, about 0.15 to about 0.75 nM and about 0.18 to about 0.72 nM. In some embodiments, the binding affinity is about 2 pM, about 5 pM, about 10 pM, about 15 pM, about 20 pM, about 40 pM, or greater than about 40 pM. In one embodiment, the binding affinity is between about 2 pM and 22 pM. In other embodiments, the binding affinity is less than about 100 nM, about 50 nM, about 10 nM, about 1 nM, about 500 pM, about 100 pM, about 50 pM, about 10 pM. In some embodiment, the binding affinity is about 10 nM. In other embodiments, the binding affinity is less than about 10 nM. In other embodiments, the binding affinity is about 0.1 nM or about 0.07 nM. In other embodiments, the binding affinity is less than about 0.1 nM or less than about 0.07 nM. In other embodiments, the binding affinity is any of about 100 nM, about 50 nM, about 10 nM, about 1 nM, about 500 pM, about 100 pM, or about 50 pM to any of about 2 pM, about 5 pM, about 10 pM, about 15 pM, about 20 pM, or about 40 pM. In some embodiments, the binding affinity is any of about 100 nM, about 50 nM, about 10 nM, about 1 nM, about 500 pM, about 100 pM, or about 50 pM, or less than about 50 pM. In still other embodiments, the binding affinity is about 2 pM, about 5 pM, about 10 pM, about 15 pM, about 20 pM, about 40 pM, or greater than about 40 pM.

One way of determining binding affinity of antibodies to NGF is by measuring affinity of monofunctional Fab fragments of the antibody. To obtain monofunctional Fab fragments, an antibody (for example, IgG) can be cleaved with papain or expressed recombinantly. The affinity of an anti-NGF Fab fragment of an antibody can be determined by surface plasmon resonance (BlAcore3000™ surface plasmon resonance (SPR) system, BIAcore, INC, Piscaway NJ). CM5 chips can be activated with N-ethyl-N'-(3-dimethylaminopropyl)-carbodiinide hydrochloride (EDC) and N-hydroxysuccinimide (NHS) according to the supplier's instructions. Human NGF can be diluted into 10 mM sodium acetate pH 4.0 and injected over the activated chip at a concentration of 0.005 mg/mL. Using variable flow time across the individual chip channels, two ranges of antigen density can be achieved: 100-200 response units (RU) for detailed kinetic studies and 500-600 RU for screening assays. The chip can be blocked with ethanolamine. Regeneration studies have shown that a mixture of Pierce elution buffer (Product No. 21004, Pierce Biotechnology, Rockford IL) and 4 M NaCl (2:1) effectively removes the bound Fab while keeping the activity of hNGF on the chip for over 200 injections. HBS-EP buffer (0.01M HEPES, pH 7.4, 0.15 NaCl, 3mM EDTA, 0.005% Surfactant P29) is used as running buffer for the BIAcore assays. Serial dilutions (0.1-10x estimated K_{D}) of purified Fab samples are injected for 1 min at 100 µL/min and dissociation times of up to 2h are allowed. The concentrations of the Fab proteins are determined by ELISA and/or SDS-PAGE electrophoresis using a Fab of known concentration (as determined by amino acid analysis) as a standard. Kinetic association rates (kₒₙ) and dissociation rates (k_{off}) are obtained simultaneously by fitting the data to a 1:1 Langmuir binding model (Karlsson, R. Roos, H. Fagerstam, L. Petersson, B. (1994). Methods Enzyology 6:99-110) using the BIAevaluation program. Equilibrium dissociation constant (K_{D}) values can be calculated as k_{off}/kₒₙ. This protocol is suitable for use in determining binding affinity of an antibody to NGF of any species, including human NGF, NGF of another vertebrate (in some embodiments, mammalian) (such as mouse NGF, rat NGF, primate NGF), as well as for use with other neurotrophins, such as the related neurotrophins NT3, NT4/5, and/or BDNF.

In some embodiments, the antibody binds human NGF, and does not significantly bind an NGF from another vertebrate species (in some embodiment, mammalian). In some embodiments, the antibody binds human NGF as well as one or more NGF from another vertebrate species (in some embodiments, mammalian). In still other embodiments, the antibody binds NGF and does not significantly cross-react with other neurotrophins (such as the related neurotrophins, NT3, NT4/5, and/or BDNF). In some embodiments, the antibody binds NGF as well as at least one other neurotrophin. In some embodiments, the antibody binds to a mammalian species of NGF, such as horse or dog, but does not significantly bind to NGF from anther mammalian species.

The epitope(s) can be continuous or discontinuous. In one embodiment, the antibody binds essentially the same hNGF epitope as an antibody selected from the group consisting of MAb 911, MAb 912, and MAb 938 as described in Hongo et al., Hybridoma, 19:215-227 (2000). In another embodiment, the antibody binds essentially the same hNGF epitope as MAb 911. In still another embodiment, the antibody binds essentially the same epitope as MAb 909. Hongo *et al.,* supra. For example, the epitope may comprise one or more of: residues K32, K34 and E35 within variable region 1 (amino acids 23-35) of hNGF; residues F79 and T81 within variable region 4 (amino acids 81-88) of hNGF; residues H84 and K88 within variable region 4; residue R103 between variable region 5 (amino acids 94-98) of hNGF and the C-terminus (amino acids 111-118) of hNGF; residue E11 within pre-variable region 1 (amino acids 10-23) of hNGF; Y52 between variable region 2 (amino acids 40-49) of hNGF and variable region 3 (amino acids 59-66) of hNGF; residues L112 and S113 within the C-terminus of hNGF; residues R59 and R69 within variable region 3 of hNGF; or residues V18, V20, and G23 within pre-variable region 1 of hNGF. In addition, an epitope can comprise one or more of the variable region 1, variable region 3, variable region 4, variable region 5, the N-terminus region, and /or the C-terminus of hNGF. In still another embodiment, the antibody significantly reduces the solvent accessibility of residue R103 of hNGF. It is understood that although the epitopes described above relate to human NGF, one of ordinary skill can align the structures of human NGF with the NGF of other species and identify likely counterparts to these epitopes.

In one aspect, antibodies (e.g., human, humanized, mouse, chimeric) that can inhibit NGF may be made by using immunogens that express full length or partial sequence of NGF. In another aspect, an immunogen comprising a cell that overexpresses NGF may be used. Another example of an immunogen that can be used is NGF protein that contains full-length NGF or a portion of the NGF protein.

The anti-NGF antibodies may be made by any method known in the art. The route and schedule of immunization of the host animal are generally in keeping with established and conventional techniques for antibody stimulation and production, as further described herein. General techniques for production of human and mouse antibodies are known in the art and are described herein.

It is contemplated that any mammalian subject including humans or antibody producing cells therefrom can be manipulated to serve as the basis for production of mammalian, including human, hybridoma cell lines. Typically, the host animal is inoculated intraperitoneally, intramuscularly, orally, subcutaneously, intraplantar, and/or intradermally with an amount of immunogen, including as described herein.

Hybridomas can be prepared from the lymphocytes and immortalized myeloma cells using the general somatic cell hybridization technique of Kohler, B. and Milstein, C. (1975) Nature 256:495-497 or as modified by Buck, D. W., et al., In Vitro, 18:377-381 (1982). Available myeloma lines, including but not limited to X63-Ag8.653 and those from the Salk Institute, Cell Distribution Center, San Diego, Calif., USA, may be used in the hybridization. Generally, the technique involves fusing myeloma cells and lymphoid cells using a fusogen such as polyethylene glycol, or by electrical means well known to those skilled in the art. After the fusion, the cells are separated from the fusion medium and grown in a selective growth medium, such as hypoxanthine-aminopterin-thymidine (HAT) medium, to eliminate unhybridized parent cells. Any of the media described herein, supplemented with or without serum, can be used for culturing hybridomas that secrete monoclonal antibodies. As another alternative to the cell fusion technique, EBV immortalized B cells may be used to produce the anti-NGF monoclonal antibodies of the subject invention. The hybridomas are expanded and subcloned, if desired, and supernatants are assayed for anti-immunogen activity by conventional immunoassay procedures (e.g., radioimmunoassay, enzyme immunoassay, or fluorescence immunoassay).

Hybridomas that may be used as source of antibodies encompass all derivatives, progeny cells of the parent hybridomas that produce monoclonal antibodies specific for NGF, or a portion thereof.

Hybridomas that produce such antibodies may be grown in vitro or in vivo using known procedures. The monoclonal antibodies may be isolated from the culture media or body fluids, by conventional immunoglobulin purification procedures such as ammonium sulfate precipitation, gel electrophoresis, dialysis, chromatography, and ultrafiltration, if desired. Undesired activity, if present, can be removed, for example, by running the preparation over adsorbents made of the immunogen attached to a solid phase and eluting or releasing the desired antibodies off the immunogen. Immunization of a host animal with a human NGF, or a fragment containing the target amino acid sequence conjugated to a protein that is immunogenic in the species to be immunized, e.g., keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glutaradehyde, succinic anhydride, SOCl2, or R1N=C=NR, where R and R1 are different alkyl groups, can yield a population of antibodies (e.g., monoclonal antibodies).

If desired, the anti-NGF antibody (monoclonal or polyclonal) of interest may be sequenced and the polynucleotide sequence may then be cloned into a vector for expression or propagation. The sequence encoding the antibody of interest may be maintained in vector in a host cell and the host cell can then be expanded and frozen for future use. In an alternative, the polynucleotide sequence may be used for genetic manipulation to "humanize" the antibody or to improve the affinity, or other characteristics of the antibody. For example, the constant region may be engineered to more resemble human constant regions to avoid immune response if the antibody is used in clinical trials and treatments in humans. It may be desirable to genetically manipulate the antibody sequence to obtain greater affinity to NGF and greater efficacy in inhibiting NGF. It will be apparent to one of skill in the art that one or more polynucleotide changes can be made to the anti-NGF antibody and still maintain its binding ability to NGF.

There are four general steps to humanize a monoclonal antibody. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. *See,* for example, U.S. Patent Nos. 4,816,567; 5,807,715; 5,866,692; 6,331,415; 5,530,101; 5,693,761; 5,693,762; 5,585,089; and 6,180,370.

A number of "humanized" antibody molecules comprising an antigen-binding site derived from a non-human immunoglobulin have been described, including chimeric antibodies having rodent or modified rodent V regions and their associated complementarity determining regions (CDRs) fused to human constant domains. *See,* for example, Winter et al. Nature 349:293-299 (1991), Lobuglio et al. Proc. Nat. Acad. Sci. USA 86:4220-4224 (1989), Shaw et al. J Immunol. 138:4534-4538 (1987), and Brown et al. Cancer Res. 47:3577-3583 (1987). Other references describe rodent CDRs grafted into a human supporting framework region (FR) prior to fusion with an appropriate human antibody constant domain. *See,* for example, Riechmann et al. Nature 332:323-327 (1988), Verhoeyen et al. Science 239:1534-1536 (1988), and Jones et al. Nature 321:522-525 (1986). Another reference describes rodent CDRs supported by recombinantly veneered rodent framework regions. *See,* for example, European Patent Publication No. 519,596. These "humanized" molecules are designed to minimize unwanted immunological response toward rodent anti-human antibody molecules which limits the duration and effectiveness of therapeutic applications of those moieties in human recipients. Other methods of humanizing antibodies that may also be utilized are disclosed by Daugherty et al., Nucl. Acids Res. 19:2471-2476 (1991) and in U.S. Patent Nos. 6,180,377; 6,054,297; 5,997,867; 5,866,692; 6,210,671; 6,350,861; and PCT Publication No. WO 01/27160.

In yet another alternative, fully human antibodies may be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are Xenomouse™ from Abgenix, Inc. (Fremont, CA) and HuMAb-Mouse® and TC Mouse™ from Medarex, Inc. (Princeton, NJ).

It is apparent that although the above discussion pertains to humanized antibodies, the general principles discussed are applicable to customizing antibodies for use, for example, in dogs, cats, primate, equines and bovines. It is further apparent that one or more aspects of humanizing an antibody described herein may be combined, e.g., CDR grafting, framework mutation and CDR mutation.

In an alternative, antibodies may be made recombinantly and expressed using any method known in the art. In another alternative, antibodies may be made recombinantly by phage display technology. *See,* for example, U.S. Patent Nos. 5,565,332; 5,580,717; 5,733,743; 6,265,150; and Winter et al., Annu. Rev. Immunol. 12:433-455 (1994). Alternatively, the phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for review *see,* e.g., Johnson, Kevin S. and Chiswell, David J., Current Opinion in Structural Biology 3, 564-571 (1993). Several sources of V-gene segments can be used for phage display. Clackson et al., Nature 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Mark et al., J. Mol. Biol. 222:581-597 (1991), or Griffith et al., EMBO J. 12:725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling." Marks, et al., Bio/Technol. 10:779-783 (1992)). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the pM-nM range. A strategy for making very large phage antibody repertoires (also known as "the mother-of-all libraries") has been described by Waterhouse et al., Nucl. Acids Res. 21:2265-2266 (1993). Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable regions capable of restoring a functional antigen-binding site, i.e., the epitope governs (imprints) the choice of partner. When the process is repeated in order to replace the remaining rodent V domain, a human antibody is obtained (*see* PCT Patent Application WO 9306213, published April 1, 1993). Unlike traditional humanization of rodent antibodies by CDR grafting, this technique provides completely human antibodies, which have no framework or CDR residues of rodent origin. It is apparent that although the above discussion pertains to humanized antibodies, the general principles discussed are applicable to customizing antibodies for use, for example, in dogs, cats, primate, equines and bovines.

Antibodies may be made recombinantly by first isolating the antibodies and antibody producing cells from host animals, obtaining the gene sequence, and using the gene sequence to express the antibody recombinantly in host cells (e.g., CHO cells). Another method which may be employed is to express the antibody sequence in plants (e.g., tobacco) or transgenic milk. Methods for expressing antibodies recombinantly in plants or milk have been disclosed. See, for example, Peeters, et al. Vaccine 19:2756 (2001); Lonberg, N. and D. Huszar Int.Rev.Immunol 13:65 (1995); and Pollock, et al., J Immunol Methods 231:147(1999). Methods for making derivatives of antibodies, e.g., humanized, single chain, etc. are known in the art.

Immunoassays and flow cytometry sorting techniques such as fluorescence activated cell sorting (FACS) can also be employed to isolate antibodies that are specific for NGF.

The antibodies can be bound to many different carriers. Carriers can be active and/or inert. Examples of well-known carriers include polypropylene, polystyrene, polyethylene, dextran, nylon, amylases, glass, natural and modified celluloses, polyacrylamides, agaroses and magnetite. The nature of the carrier can be either soluble or insoluble for purposes of the invention. Those skilled in the art will know of other suitable carriers for binding antibodies, or will be able to ascertain such, using routine experimentation.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors (such as expression vectors disclosed in WO87/04462), which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. See, e.g., PCT Publication No. WO87/04462. The DNA also may be modified, for example, by substituting the coding sequence for human heavy and light chain constant domains in place of the homologous murine sequences, Morrison et al., Proc. Nat. Acad. Sci. 81:6851 (1984), or by covalently joining to the immunoglobulin coding sequence all or part of the coding sequence for a non-immunoglobulin polypeptide. In that manner, "chimeric" or "hybrid" antibodies are prepared that have the binding specificity of an anti-NGF monoclonal antibody herein.

Anti-NGF antibodies may be characterized using methods well known in the art. For example, one method is to identify the epitope to which it binds, or "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, New York, 1999. In an additional example, epitope mapping can be used to determine the sequence to which an anti-NGF antibody binds. Epitope mapping is commercially available from various sources, for example, Pepscan Systems (Edelhertweg 15, 8219 PH Lelystad, The Netherlands). The epitope can be a linear epitope, i.e., contained in a single stretch of amino acids, or a conformational epitope formed by a three-dimensional interaction of amino acids that may not necessarily be contained in a single stretch. Peptides of varying lengths (e.g., at least 4-6 amino acids long) can be isolated or synthesized (e.g., recombinantly) and used for binding assays with an anti-NGF antibody. In another example, the epitope to which the anti-NGF antibody binds can be determined in a systematic screening by using overlapping peptides derived from the NGF sequence and determining binding by the anti-NGF antibody. According to the gene fragment expression assays, the open reading frame encoding NGF is fragmented either randomly or by specific genetic constructions and the reactivity of the expressed fragments of NGF with the antibody to be tested is determined. The gene fragments may, for example, be produced by PCR and then transcribed and translated into protein in vitro, in the presence of radioactive amino acids. The binding of the antibody to the radioactively labeled NGF fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays. In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant NGF in which various fragments of the NGF polypeptide have been replaced (swapped) with sequences from a closely related, but antigenically distinct protein (such as another member of the neurotrophin protein family). By assessing binding of the antibody to the mutant NGF, the importance of the particular NGF fragment to antibody binding can be assessed.

Yet another method which can be used to characterize an anti-NGF antibody is to use competition assays with other antibodies known to bind to the same antigen, i.e., various fragments on NGF, to determine if the anti-NGF antibody binds to the same epitope as other antibodies. Competition assays are well known to those of skill in the art. Example of antibodies that can be used in the competition assays for the present invention include MAb 911, 912, 938, as described in Hongo, et al., Hybridoma 19:215-227 (2000).

### Other NGF antagonists

NGF antagonists other than anti-NGF antibodies are also described herein. In some aspects, the NGF antagonist comprises at least one antisense molecule capable of blocking or decreasing the expression of a functional NGF. Nucleotide sequences of the NGF are known and are readily available from publicly available databases. *See,* e.g., Borsani et al., Nuc. Acids Res. 1990, 18, 4020; Accession Number NM 002506; Ullrich et al., Nature 303:821-825 (1983). It is routine to prepare antisense oligonucleotide molecules that will specifically bind NGF mRNA without cross-reacting with other polynucleotides. Exemplary sites of targeting include, but are not limited to, the initiation codon, the 5' regulatory regions, the coding sequence and the 3' untranslated region. In some aspects, the oligonucleotides are about 10 to 100 nucleotides in length, about 15 to 50 nucleotides in length, about 18 to 25 nucleotides in length, or more. The oligonucleotides can comprise backbone modifications such as, for example, phosphorothioate linkages, and 2'-O sugar modifications well know in the art. Exemplary antisense molecules include the NGF antisense molecules described in U.S. Publication No. 20010046959; *see* also http://www.rna-tec.com/repair.htm.

In other aspects, the NGF antagonist comprises at least one antisense molecule capable of blocking or decreasing the expression of a functional NGF receptor (such as TrkA and/or p75). Woolf et al., J. Neuroscie (2001)21(3):1047-55; Taglialetela et al, JNeurochem (1996) 66(5): 1826-35. Nucleotide sequences of TrkA and p75 are known and are readily available from publicly available databases.

Alternatively, NGF expression and/or release can be decreased using gene knockdown, morpholino oligonucleotides, RNAi, or ribozymes, methods that are well-known in the art. *See* http://www.macalester.edu/∼montgomery/RNAi.html; http://pub32.ezboard.com/fmorpholinosfrm19.showMessage?topicID=6.topic; http://www.highveld.com/ribozyme.html.

In other aspects, the NGF antagonist comprises at least one NGF inhibitory compound. As used herein, "NGF inhibitory compound" refers to a compound other than an anti-NGF antibody that directly or indirectly reduces, inhibits, neutralizes, or abolishes NGF biological activity. An NGF inhibitory compound should exhibit any one or more of the following characteristics: (a) bind to NGF; (b) inhibit NGF biological activity and/or downstream pathways mediated by NGF signaling function; (c) treating or preventing any aspect of pain, particularly in conjunction with an opioid analgesic; (d) block or decrease NGF receptor activation (including trkA receptor dimerization and/or autophosphorylation); (e) increase clearance of NGF; (f) inhibit (reduce) NGF synthesis, production or release; (g) enhance opioid treatment of pain. Exemplary NGF inhibitory compounds include the small molecule NGF inhibitors described in U.S. Publication No. 20010046959; the compounds that inhibit NGF's binding to p75, as described in PCT Publication No. WO 00/69829; the compounds that inhibit NGF's binding to TrkA/p75, as described in PCT Publication No. WO 98/17278. Additional examples of NGF inhibitory compounds include the compounds described in PCT Publication Nos. WO 02/17914, WO 02/20479, U.S. Patent No. 5,342,942, 6,127,401, and 6,359,130. Further exemplary NGF inhibitory compounds are compounds that are competitive inhibitors of NGF. *See* U.S. Patent No. 6,291,247. Furthermore, one skilled in the art can prepare other small molecules NGF inhibitory compounds.

In some aspects, an NGF inhibitory compound binds NGF. Exemplary sites of targeting (binding) include, but are not limited to, the portion of the NGF that binds to the TrkA and/or p75 receptor, and those portions of the NGF that are adjacent to the receptor-binding region and which are responsible, in part, for the correct three-dimensional shape of the receptor-binding portion. In another embodiment, an NGF inhibitory compound binds an NGF receptor (such as TrkA and/or p75) and inhibits an NGF biological activity. Exemplary sites of targeting include those portions of TrkA and/or p75 that bind to NGF.

In aspects comprising small molecules, a small molecule can have a molecular weight of about any of 100 to 20,000 daltons, 500 to 15,000 daltons, or 1000 to 10,000 daltons. Libraries of small molecules are commercially available. The small molecules can be administered using any means known in the art, including inhalation, intraperitoneally, intravenously, intramuscularly, subcutaneously, intrathecally, intraventricularly, orally, enterally, parenterally, intranasally, or dermally. In some aspects, when the NGF antagonist is a small molecule, it will be administered at the rate of 0.1 to 300 mg/kg of the weight of the patient divided into one to three or more doses. For an adult patient of normal weight, doses ranging from 1 mg to 5g per dose can be administered.

In other aspects, the NGF antagonist comprises at least one NGF structural analog. "NGF structural analogs" refer to compounds that have a similar 3-dimensional structure as part of that of NGF and which bind to an NGF receptor under physiological conditions *in vitro or in vivo.* In one aspect, the NGF structural analog binds to a TrkA and/or a p75 receptor. Exemplary NGF structural analogs include, but are not limited to, the bicyclic peptides described in PCT Publication No. WO 97/15593; the bicyclic peptides described in U.S. Patent No. 6,291,247; the cyclic compounds described in U.S. Patent No. 6,017,878; and NGF-derived peptides described in PCT Publication No. WO 89/09225. Suitable NGF structural analogs can also be designed and synthesized through molecular modeling of NGF-receptor binding, for example by the method described in PCT Publication No. WO 98/06048. The NGF structural analogs can be monomers or dimers/oligomers in any desired combination of the same or different structures to obtain improved affinities and biological effects.

In other aspects, the NGF antagonist comprises at least one dominant-negative mutant of the TrkA and/or p75 receptor. One skilled in the art can prepare dominant-negative mutants of, e.g., the TrkA receptor such that the receptor will bind the NGF and, thus, act as a "sink" to capture NGFs. The dominant-negative mutants, however, will not have the normal bioactivity of the receptor (such as TrkA receptor) upon binding to NGF. Exemplary dominant-negative mutants include, but are not limited to, the mutants described in the following references: Li et al., Proc. Natl. Acad. Sci. USA 1998, 95, 10884; Eide et al., J. Neurosci. 1996, 16, 3123; Liu et al., J. Neurosci 1997, 17, 8749; Klein et al., Cell 1990, 61, 647; Valenzuela et al., Neuron 1993, 10, 963; Tsoulfas et al., Neuron 1993, 10, 975; and Lamballe et al., EMBO J. 1993, 12, 3083. The dominant negative mutants can be administered in protein form or in the form of an expression vector such that dominant-negative mutant (e.g., a mutant TrkA receptor) is expressed in vivo. The protein or expression vector can be administered using any means known in the art, such as intraperitoneally, intravenously, intramuscularly, subcutaneously, intrathecally, intraventricularly, orally, enterally, parenterally, intranasally, dermally, or by inhalation. For example, administration of expression vectors includes local or systemic administration, including injection, oral administration, particle gun or catheterized administration, and topical administration. One skilled in the art is familiar with administration of expression vectors to obtain expression of an exogenous protein in vivo. *See,* e.g., U.S. Patent Nos. 6,436,908; 6,413,942; 6,376,471.

Targeted delivery of therapeutic compositions containing an antisense polynucleotide, expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655; Wu et al., J. Biol. Chem. (1991) 266:338. Therapeutic compositions containing a polynucleotide are administered in a range of about 100 ng to about 200 mg (or more) of DNA for local administration in a gene therapy protocol. In some aspects, concentration ranges of less than about 500 ng, about 500 ng to about 50 mg, about 1 µg to about 2 mg, about 5 µg to about 500 µg, and about 20 µg to about 100 µg or more of DNA can also be used during a gene therapy protocol. The therapeutic polynucleotides and polypeptides of the present disclosure can be delivered using gene delivery vehicles. The gene delivery vehicle can be of viral or non-viral origin *(see* generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters and/or enhancers. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (*see,* e.g., PCT Publication Nos. WO 90/07936; WO 94/03622; WO 93/25698; WO 93/25234; WO 93/11230; WO 93/10218; WO 91/02805; U.S. Patent Nos. 5, 219,740; 4,777,127; GB Patent No. 2,200,651; and EP Patent No. 0 345 242), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors *(see,* e.g., PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone *(see,* e.g., Curiel, Hum. Gene Ther. (1992) 3:147); ligand-linked DNA(*see*, e.g., Wu, J. Biol. Chem. (1989) 264:16985); eukaryotic cell delivery vehicles cells *(see,* e.g., U.S. Patent No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Patent No. 5,580,859. Liposomes that can act as gene delivery vehicles are described in U.S. Patent No. 5,422,120; PCT Publication Nos. WO 95/13796; WO 94/23697; WO 91/14445; and EP Patent No. 0 524 968. Additional approaches are described in Philip, Mol. Cell Biol. (1994) 14:2411, and in Woffendin, Proc. Natl. Acad. Sci. (1994) 91:1581.

It is also apparent that an expression vector can be used to direct expression of any of the protein-based NGF antagonists described herein (e.g., anti-NGF antibody, TrkA immunoadhesin, etc.). For example, other TrkA receptor fragments that are capable of blocking (from partial to complete blocking) NGF and/or an NGF biological activity are known in the art.

In another aspect, the NGF antagonist comprises at least one TrkA immunoadhesin. TrkA immunoadhesins as used herein refer to soluble chimeric molecules comprising the extracellular domain of a TrkA receptor (or a portion thereof) and an immunoglobulin sequence, which retains the binding specificity (in some embodiments, substantially retains the binding specificity) of the TrkA receptor and is capable of binding to NGF. A trkA immunoadhesin is capable of blocking (reducing and/or suppressing) a NGF biological activity, as described herein.

TrkA immunoadhesins are known in the art, and have been found to block (reduce or suppress) the binding of NGF to the TrkA receptor. *See,* e.g., U.S. Patent No. 6,153,189. In one aspect, the TrkA immunoadhesin comprises a fusion of a TrkA receptor amino acid sequence capable of binding NGF (or an amino acid sequence that substantially retains the binding specificity of the trkA receptor) and an immunoglobulin sequence (or an amino acid that substantially retains the binding specificity of the TrkA receptor). In some aspects, the TrkA receptor is a human TrkA receptor sequence, and the fusion is with an immunoglobulin constant domain sequence. In other aspects, the immunoglobulin constant domain sequence is an immunoglobulin heavy chain constant domain sequence. In other aspects, the association of two TrkA receptor-immunoglobulin heavy chain fusions (e.g., via covalent linkage by disulfide bond(s)) results in a homodimeric immunoglobulin-like structure. An immunoglobulin light chain can further be associated with one or both of the TrkA receptor-immunoglobulin chimeras in the disulfide-bonded dimer to yield a homotrimeric or homotetrameric structure. Examples of suitable TrkA immunoadhesins include those described in U.S. Patent No. 6,153,189.

In another aspect, the NGF antagonist comprises at least one anti-TrkA antibody capable of blocking, suppressing, altering, and/or reducing NGF physical interaction with the TrkA receptor and/or downstream signaling, whereby an NGF biological activity is reduced and/or blocked. Anti-TrkA antibodies are known in the art. Exemplary anti-TrkA antibodies include those described in PCT Publication Nos. WO 97/21732, WO 00/73344, WO 02/15924, and U.S. Publication No. 20010046959. In another aspect, the NGF antagonist comprises at least one anti-p75 antibody capable of blocking, suppressing and/or reducing NGF physical interaction with the p75 receptor and/or downstream signaling, whereby an NGF biological activity is reduced and/or blocked.

In another aspect, the NGF antagonist comprises at least one kinase inhibitor capable of inhibiting downstream kinase signaling associated with TrkA and/or p75 receptor activity. An exemplary kinase inhibitor is K252a or K252b, which is known in the art and described in Knusel et al., J. Neurochem. 59:715-722 (1992); Knusel et al., J. Neurochemistry 57:955-962 (1991); Koizumi et al., J. Neuroscience 8:715-721 (1988); Hirata et al., Chemical Abstracts 111:728, XP00204135, *see* abstract and 12th Collective Chemical Substance Index, p. 34237, c. 3 (5-7), 55-60, 66-69), p. 34238, c.1 (41-44), c.2 (25-27, 32-33), p. 3423, c.3 (48-50, 52-53); U.S. Patent No. 6,306,849.

It is expected that a number of other categories of NGF antagonists will be identified if sought for by the clinician.

### Identification of NGF antagonists

Anti-NGF antibodies and other NGF antagonists can be identified or characterized using methods known in the art, whereby reduction, amelioration, or neutralization of an NGF biological activity is detected and/or measured. For example, a kinase receptor activation (KIRA) assay described in U.S. Patent No. 5,766,863 and 5,891,650, can be used to identify NGF antagonists. This ELISA-type assay is suitable for qualitative or quantitative measurement of kinase activation by measuring the autophosphorylation of the kinase domain of a receptor protein tyrosine kinase (hereinafter "rPTK"), e.g. TrkA receptor, as well as for identification and characterization of potential antagonists of a selected rPTK, e.g., TrkA. The first stage of the assay involves phosphorylation of the kinase domain of a kinase receptor, for example, a TrkA receptor, wherein the receptor is present in the cell membrane of an eukaryotic cell. The receptor may be an endogenous receptor or nucleic acid encoding the receptor, or a receptor construct, may be transformed into the cell. Typically, a first solid phase (e.g., a well of a first assay plate) is coated with a substantially homogeneous population of such calls (usually a mammalian cell line) so that the cells adhere to the solid phase. Often, the cells are adherent and thereby adhere naturally to the first solid phase. If a "receptor construct" is used, it usually comprises a fusion of a kinase receptor and a flag polypeptide. The flag polypeptide is recognized by the capture agent, often a capture antibody, in the ELISA part of the assay. An analyte, such as a candidate anti-NGF antibody or other NGF antagonist, is then added together with NGF to the wells having the adherent cells, such that the tyrosine kinase receptor (e.g. TrkA receptor) is exposed to (or contacted with) NGF and the analyte. This assay enables identification antibodies (or other NGF antagonist) that inhibit activation of TrkA by its ligand NGF. Following exposure to NGF and the analyte, the adhering cells are solubilized using a lysis buffer (which has a solubilizing detergent therein) and gentle agitation, thereby releasing cell lysate which can be subjected to the ELISA part of the assay directly, without the need for concentration or clarification of the cell lysate.

The cell lysate thus prepared is then ready to be subjected to the ELISA stage of the assay. As a first step in the ELISA stage, a second solid phase (usually a well of an ELISA microtiter plate) is coated with a capture agent (often a capture antibody) which binds specifically to the tyrosine kinase receptor, or, in the case of a receptor construct, to the flag polypeptide. Coating of the second solid phase is carried out so that the capture agent adheres to the second solid phase. The capture agent is generally a monoclonal antibody, but, as is described in the examples herein, polyclonal antibodies may also be used. The cell lysate obtained is then exposed to, or contacted with, the adhering capture agent so that the receptor or receptor construct adheres to (or is captured in) the second solid phase. A washing step is then carried out, so as to remove unbound cell lysate, leaving the captured receptor or receptor construct. The adhering or captured receptor or receptor construct is then exposed to, or contacted with, an anti-phosphotyrosine antibody which identifies phosphorylated tyrosine residues in the tyrosine kinase receptor. In one embodiment, the anti-phosphotyrosine antibody is conjugated (directly or indirectly) to an enzyme which catalyses a color change of a non-radioactive color reagent. Accordingly, phosphorylation of the receptor can be measured by a subsequent color change of the reagent. The enzyme can be bound to the anti-phosphotyrosine antibody directly, or a conjugating molecule (e.g., biotin) can be conjugated to the anti-phosphotyrosine antibody and the enzyme can be subsequently bound to the anti-phosphotyrosine antibody via the conjugating molecule. Finally, binding of the anti-phosphotyrosine antibody to the captured receptor or receptor construct is measured, e.g., by a color change in the color reagent.

The NGF antagonist can also be identified by incubating a candidate agent with NGF and monitoring any one or more of the following characteristics: (a) binding to NGF; (b) inhibiting NGF biological activity or downstream pathways mediated by NGF signaling function; (c) blocking or decreasing NGF receptor activation (including TrkA dimerization and/or autophosphorylation); (d) increasing clearance of NGF; (e) treating, ameliorating or preventing any aspect of pain, particularly in conjunction with an opioid analgesic; (f) inhibit (reduce) NGF synthesis, production or release; (g) enhance opioid treatment of pain. In some embodiments, an NGF antagonist is identified by incubating an candidate agent with NGF and monitoring binding and attendant reduction or neutralization of a biological activity of NGF. The binding assay may be performed with purified NGF polypeptide(s), or with cells naturally expressing, or transfected to express, NGF polypeptide(s). In one embodiment, the binding assay is a competitive binding assay, where the ability of a candidate antibody to compete with a known NGF antagonist for NGF binding is evaluated. The assay may be performed in various formats, including the ELISA format. In other embodiments, an NGF antagonist is identified by incubating a candidate agent with NGF and monitoring attendant inhibition of TrkA receptor dimerization and/or autophosphorylation.

Following initial identification, the activity of a candidate anti-NGF antagonist can be further confirmed and refined by bioassays, known to test the targeted biological activities. Alternatively, bioassays can be used to screen candidates directly. For example, NGF promotes a number of morphologically recognizable changes in responsive cells. These include, but are not limited to, promoting the differentiation of PC12 cells and enhancing the growth of neurites from these cells (Urfer et al., Biochem. 36:4775-4781 (1997); Tsoulfas et al., Neuron 10:975-990 (1993)), promoting neurite outgrowth from explants of responsive sensory and sympathetic ganglia (Levi-Montalcini, R. and Angeletti, P. Nerve growth factor. Physiol. Rev. 48, 534-569, 1968) and promoting the survival of NGF dependent neurons such as embryonic dorsal root ganglion, trigeminal ganglion, or sympathetic ganglion neurons (e.g., Chun & Patterson, Dev. Biol. 75:705-711, (1977); Buchman & Davies, Development 118:989-1001, (1993). Thus, the assay for inhibition of NGF biological activity entail culturing NGF responsive cells with NGF plus an analyte, such as a candidate anti-NGF antibody or a candidate NGF antagonist. After an appropriate time the cell response will be assayed (cell differentiation, neurite outgrowth or cell survival).

The ability of a candidate NGF antagonist to block or neutralize a biological activity of NGF can also be assessed by monitoring the ability of the candidate agent to inhibit NGF mediated survival in the embryonic rat dorsal root ganglia survival bioassay as described in Hongo et al., Hybridoma 19:215-227 (2000).

### Compositions

The compositions for use in the invention comprise an effective amount of an anti-NGF antibody and an opioid analgesic, as described in various embodiments herein. In some embodiments, the compositions further comprise a pharmaceutically acceptable excipient. In some aspects, the composition is for use in any of the methods described herein (such as methods for treating post-surgical pain). Examples of such compositions, as well as how to formulate, are also described in an earlier section and below. The anti-NGF antibody and opioid may be present in a single composition or present as separate compositions. Accordingly, in some embodiments, the anti-NGF antibody and the opioid analgesic are present in the same composition. In other embodiments, the anti-NGF antibody and opioid analgesic are present in separate compositions.

In another aspect, the disclosure provides a synergistic composition of an anti-NGF antibody and an opioid analgesic.

In some embodiments, the invention provides pharmaceutical compositions comprising an anti-NGF antibody for use in the treatment of post-surgical pain, wherein said use comprises simultaneous and/or sequential administration of an opioid analgesic. In some embodiments, the invention provides pharmaceutical compositions comprising an opioid analgesic for use in the treatment of post-surgical pain, wherein said use comprises simultaneous and/or sequential administration of an anti-NGF antibody. In some embodiments, the invention provides pharmaceutical compositions comprising an anti-NGF antibody and an opioid analgesic for separate, simultaneous and/or sequential use for treatment of post-surgical pain. In some embodiments, the anti-NGF antibody is antibody E3 as described herein. In other embodiments, the opioid analgesic is morphine. It is understood that the compositions can comprise more than one NGF antagonist. For example, a composition can comprise more than one member of a class of NGF antagonist (e.g., a mixture of anti-NGF antibodies that recognize different epitopes of NGF), as well as members of different classes of NGF antagonists (e.g., an anti-NGF antibody and an NGF inhibitory compound). Other exemplary compositions comprise more than one anti-NGF antibody that recognizes the same epitope(s), different species of anti-NGF antibodies that bind to different epitopes of NGF, or different NGF inhibitory compounds. In other aspects, the composition comprises one or more NGF antagonists selected from the group consisting of an antagonist (e.g., an antibody) that binds (physically interacts with) NGF, an antagonist that binds to an NGF receptor (such as the TrkA receptor or the p75 receptor), and/or an antagonist that reduces (impedes and/or blocks) downstream NGF receptor signaling.

The composition used in the present invention can further comprise pharmaceutically acceptable carriers, excipients, or stabilizers (Remington: The Science and Practice of Pharmacy 20th Ed. (2000) Lippincott Williams and Wilkins, Ed. K. E. Hoover), in the form of lyophilized formulations or aqueous solutions. Acceptable carriers, excipients, or stabilizers are nontoxic to recipients at the dosages and concentrations, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG). Pharmaceutically acceptable excipients are further described herein.

The compositions described herein may contain additional compounds known to be useful for the treatment of pain. The NGF antagonist and opioid analgesic, and compositions thereof can also be used in conjunction with other agents that serve to enhance and/or complement the effectiveness of the agents.

### Kits

The invention also provides kits for use in the instant medical uses. The invention provides a kit for use in treating post-surgical pain comprising an NGF antagonist, wherein the NGF antagonist is administered simultaneously, separately or sequentially with an opioid analgesic for the effective treatment of post-surgical pain; wherein the NGF antagonist is an anti-NGF antibody which inhibits the binding of NGF to its trkA and p75 receptor *in vivo.* Kits for use in the invention include one or more containers comprising an anti-NGF antibody, such as humanized antibody E3 described herein and/or an opioid analgesic, and in some embodiments, further comprise instructions for use in accordance with any of the medical uses for treating post-surgical pain described herein. In other embodiments, the kit comprises an anti-NGF antibody comprising one or more CDR(s) of antibody E3 (such as one, two, three, four, five, or, in some embodiments, all six CDRs from E3). The kit may further comprise a description of selecting an individual suitable for treatment based on identifying whether that individual has post-surgical pain or whether the individual is at risk of post-surgical pain. In still other embodiments, the kit comprises a humanized antibody (such as antibody E3 described herein). In still other embodiments, the instructions comprise description of administering an anti-NGF antibody in conjunction with an opioid analgesic to treat, prevent and/or ameliorate post-surgical pain.

In some embodiments, the kit comprises an anti-NGF antibody, an opioid analgesic, and instructions for administering the anti-NGF antibody and the opioid analgesic simultaneously, separately or sequentially, for the effective treatment of post-surgical pain.

In some embodiments, the kit comprises an anti-NGF antibody. In other embodiments, the anti-NGF antibody is an antibody comprising the heavy chain variable region shown in Table 1 and the light chain variable region shown in Table 2. In still other embodiments, the anti-NGF antibody is antibody E3 as described herein.

The anti-NGF antibody and opioid analgesic can be present in separate containers or in a single container. It is understood that the kit may comprise one distinct composition or two or more compositions wherein one composition comprises an anti-NGF antibody and one composition comprises an opioid analgesic.

The kits for use in this invention are in suitable packaging. Suitable packaging includes, but is not limited to, vials, bottles, jars, flexible packaging (e.g., sealed Mylar or plastic bags), and the like. Kits may optionally provide additional components such as buffers and interpretive information.

The instructions relating to the use of an anti-NGF antibody generally include information as to dosage, dosing schedule, and route of administration for the intended treatment. The containers may be unit doses, bulk packages (e.g., multi-dose packages) or sub-unit doses. Instructions supplied in the kits of the invention are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating, ameliorating and/or preventing post-surgical pain. Instructions may be provided for practicing any of the methods described herein.

The kits for use in this invention are in suitable packaging. Suitable packaging includes, but is not limited to, vials, bottles, jars, flexible packaging (e.g., sealed Mylar or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an anti-NGF antibody. The container may further comprise a second pharmaceutically active agent.

Kits may optionally provide additional components such as buffers and interpretive information. Normally, the kit comprises a container and a label or package insert(s) on or associated with the container.

### Administration of an NGF antagonist and opioid analgesic, and assessment of treatment

The anti-NGF antibody and opioid analgesic can be administered to an individual via any suitable route. For example, they can be administered together or separately orally, intravenously, sublingually, subcutaneously, intraarterially, intramuscularly, rectally, intraspinally, intrathoracically, intraperitoneally, intraventricularly, sublingually, transdermally or by inhalation. They can be administered orally, for example, in the form of tablets, troches, capsules, elixirs, suspensions, syrups, wafers, chewing gum, lolliopops, suppositories or the like prepared by art recognized procedures. It will be apparent to a person skilled in the art that the examples described herein are not intended to be limiting but to be illustrative of the techniques available.

Accordingly, in some embodiments, the anti-NGF antibody, is administered to a individual in accordance with known methods, such as intravenous administration, e.g., as a bolus or by continuous infusion over a period of time, by intramuscular, intraperitoneal, intracerebrospinal, subcutaneous, intra-articular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution. Alternatively, NGF antagonist can be aerosolized using a fluorocarbon formulation and a metered dose inhaler, or inhaled as a lyophilized and milled powder.

Site-specific or targeted local delivery techniques are also useful for administration. Examples of site-specific or targeted local delivery techniques include various implantable depot sources of the NGF antagonist and/or opioid analgesic, or local delivery catheters, such as infusion catheters, an indwelling catheter, or a needle catheter, synthetic grafts, adventitial wraps, shunts and stents or other implantable devices, site specific carriers, direct injection, use of a patient controlled analgesia (PCA) technique or device, and/or direct application. *See,* e.g., PCT Publication No. WO 00/53211 and U.S. Patent No. 5,981,568.

Various formulations of NGF antagonists such as an anti-NGF antibody may be used for administration. In some embodiments, an NGF antagonist may be administered neat. In some embodiments, the NGF antagonist comprises an anti-NGF antibody, and may be in various formulations, including formulations comprising a pharmaceutically acceptable excipient. Pharmaceutically acceptable excipients are known in the art, and are relatively inert substances that facilitate administration of a pharmacologically effective substance. For example, an excipient can give form or consistency, or act as a diluent. Suitable excipients include but are not limited to stabilizing agents, wetting and emulsifying agents, salts for varying osmolarity, encapsulating agents, buffers, and skin penetration enhancers. Excipients as well as formulations for parenteral and nonparenteral drug delivery are set forth in Remington, et al., The Science and Practice of Pharmacy 20th Ed. Mack Publishing (2000).

In some embodiments, the NGF antagonist are formulated for administration by injection (e.g., intraperitoneally, intravenously, subcutaneously, intramuscularly, etc.). Accordingly, these agents can be combined with pharmaceutically acceptable vehicles such as saline, Ringer's solution, dextrose solution, and the like. The particular dosage regimen, i.e., dose, timing and repetition, will depend on the particular individual and that individual's medical history. The dosing regimen (including the NGF antagonist(s) used) can vary over time.

An anti-NGF antibody can be administered using any suitable method, including by injection (e.g., intraperitoneally, intravenously, subcutaneously, intramuscularly, etc.). An anti-NGF antibody can also be administered via inhalation, as described herein. Generally, for administration of anti-NGF antibodies, an initial candidate dosage can be about 2 mg/kg. In some embodiments, a typical daily dosage might range from any of about 3µg/kg to 30 µg/kg to 300 µg/kg to 3 mg/kg to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to reduce the pain. An exemplary dosing regimen comprises administering an initial dose of about 2 mg/kg, followed by a weekly maintenance dose of about 1 mg/kg of the anti-NGF antibody, or followed by a maintenance dose of about 1 mg/kg every other week. However, other dosage regimens may be useful, depending on the pattern of pharmacokinetic decay that the practitioner wishes to achieve. For example, dosing from one-four time a week is contemplated. Other dosing regimens include a regimen of up to 1 time per day, 1 to 4 times per week, or less frequently. In some embodiments, the compounds are administered about once per week, about 1 to 4 times per month. Dosage of anti-NGF antibodies are described herein. The progress of this therapy is easily monitored by conventional techniques and assays.

In some embodiments, when it is not an antibody, an NGF antagonist according to the disclosure may (in some embodiments) be administered at the rate of 0.1 to 300 mg/kg of the weight of the patient divided into one to three doses, or as disclosed herein. In some adult patients of normal weight, doses ranging from about 0.3 to 5.00 mg/kg may be administered. The particular dosage regimen, i.e., dose, timing and repetition, will depend on the particular individual and that individual's medical history, as well as the properties of the individual agents (such as the half-life of the agent, and other considerations well known in the art).

The opioid analgesic may be administered at a dosage level up to conventional dosage levels for such analgesics. In some embodiment, the opioid analgesic is administered at a reduced level. Suitable dosage levels will depend upon the analgesic effect of the chosen opioid analgesic, but typically suitable levels will be about 0.001 to 25 mg/kg per day, about 0.005 to 10 mg/kg per day, or about 0.05 to 1 mg/kg per day, ore less. The compound may be administered on a regimen of up to 6 times per day (or more), 1 to 4 times per day, or it may be administered less often. In some embodiments, the opioid analgesic is administered continuously, or very frequently (as with, for example PCA).

When administered in combination, either as a single or as separate composition(s), the nerve growth factor antagonist and the opioid analgesic are presented in a ratio which is consistent with the manifestation of the desired effect. In some embodiments, the ratio by weight of the nerve growth factor antagonist to the opioid analgesic will be approximately 1 to 1. In some embodiments, this ratio may be between about 0.001 to about 1 and about 1000 to about 1, or between about 0.01 to about 1 and 100 to about 1. Other ratios are contemplated.

It will be appreciated that the amount of a nerve growth factor antagonist and opioid analgesic required for use in the treatment or prevention of pain will vary not only with the particular compounds or compositions selected but also with the route of administration, the nature of the condition being treated, and the age and condition of the patient, the course or stage of treatment, and will ultimately be at the discretion of the attendant physician. For example, the appropriate dosage of an NGF antagonist (such as an anti-NGF antibody) will depend on the NGF antagonist(s) (or compositions thereof) employed, the type and severity of the pain to be treated, whether the agent is administered for preventive or therapeutic purposes, previous therapy, the patient's clinical history and response to the agent, and the discretion of the attending physician. Typically the clinician will administer an NGF antagonist, such as an anti-NGF antibody, until a dosage is reached that achieves the desired result.

Empirical considerations, such as the half-life, generally will contribute to the determination of the dosage. For example, antibodies that are compatible with the human immune system, such as humanized antibodies or fully human antibodies may be used to prolong half-life of the antibody and to prevent the antibody being attacked by the host's immune system. Frequency of administration may be determined and adjusted over the course of therapy, and is generally, but not necessarily, based on treatment and/or suppression and/or amelioration and/or delay of pain. Alternatively, sustained continuous release formulations of an NGF antagonist and/or an opioid analgesic maybe appropriate. Various formulations and devices for achieving sustained release are known in the art.

In one embodiment, dosages for an NGF antagonist may be determined empirically in individuals who have been given one or more administration(s) of an NGF antagonist to treat pain. Individuals are given incremental dosages of an NGF antagonist, i.e., anti-NGF antibody, in conjunction with opioid analgesic. To assess efficacy of the treatment, an indicator of pain can be followed.

Administration of an NGF antagonist and the opioid analgesic in accordance with the method in the present invention can be continuous or intermittent, depending, for example, upon the recipient's physiological condition, whether the purpose of the administration is therapeutic or prophylactic, and other factors known to skilled practitioners. The administration of an NGF antagonist may be essentially continuous over a preselected period of time or may be in a series of spaced dose, e.g., either before; during; or after developing pain; before and after; during and after; before and during; or before, during, and after developing pain. For example, administration can be before, during and/or after wound, incision, trauma, surgery, and any other event likely to give rise to pain.

In some embodiments, more than one NGF antagonist, such as an antibody, may be present. The antagonist can be the same or different from each other. At least one, at least two, at least three, at least four, at least five, or more different NGF antagonists can be present. Generally, those NGF antagonists have complementary activities that do not adversely affect each other. NGF antagonists can also be used in conjunction with other agents that serve to enhance and/or complement the effectiveness of the agents.

In some embodiments, more than one opioid analgesic may be present. The opioid analgesic can be the same or different from each other. At least one, at least two, at least three, at least four, at least five or more different opioid analgesic can be present. Generally, those opioid analgesics have complementary activities that do not adversely affect each other. An opioid analgesic(s) can also be used in conjunction with other agents that serve to enhance and/or complement the effectiveness of the agent(s).

Therapeutic formulations of the NGF antagonist (i.e. an antibody) and opioid analgesic used in accordance with the present invention are prepared for storage by mixing an antibody having the desired degree of purity with optional pharmaceutically acceptable carriers, excipients or stabilizers (Remington, The Science and Practice of Pharmacy 20th Ed. Mack Publishing (2000)), in the form of lyophilized formulations or aqueous solutions. Acceptable carriers, excipients, or stabilizers are nontoxic to recipients at the dosages and concentrations employed, and may comprise buffers such as phosphate, citrate, and other organic acids; salts such as sodium chloride, antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens, such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrin; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Liposomes containing the NGF antagonist (such as an antibody) are prepared by methods known in the art, such as described in Epstein, et al., Proc. Natl. Acad. Sci. USA 82:3688 (1985); Hwang, et al., Proc. Natl Acad. Sci. USA 77:4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington, The Science and Practice of Pharmacy 20th Ed. Mack Publishing (2000).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or 'poly(v nylalcohol)), polylactides (U.S. Patent No. 3,773,919), copolymers of L-glutamic acid and 7 ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), sucrose acetate isobutyrate, and poly-D-(-)-3-hydroxybutyric acid.

The formulations to be used for *in vivo* administration must be sterile. This is readily accomplished by, for example, filtration through sterile filtration membranes. For example, therapeutic anti-NGF antibody compositions are generally placed into a container having a sterile access port, for example, an intravenous solution bag or vial having a stopper pierceable by a hypodermic injection needle.

The compositions for use according to the present invention may be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound for use in the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from about 0.01 mg to about 0.1 mg to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions for use in the present invention may be incorporated for administration orally or by injection include aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin. The active ingredients may also be incorporated in highly viscous controlled release products such as sucrose acetate isobutyrate or others. These formulations may be used either for oral dosing, or injection. The injection can result in a local depot of the drug which is released locally over the course of 1 day to three months.

Compositions for administration by injection include those comprising a NGF antagonist and an opioid analgesic, as the active ingredients, in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (e.g. Tween™ 20, 40, 60, 80 or 85) and other sorbitans (e.g. Span™ 20, 40, 60, 80 or 85). Compositions with a surface-active agent will conveniently comprise between 0.05 and 5% surface-active agent, or between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn™, Infonutrol™, Lipofondin™ and Lipiphysan™ The active ingredient may be either dissolved in a pre-mixed emulsion composition or alternatively it may be dissolved in an oil (e.g. soybean oil, safflower oil, cottonseed oil, sesame oil, corn oil or almond oil) and an emulsion formed upon mixing with a phospholipid (e.g. egg phospholipids, soybean phospholipids or soybean lecithin) and water. It will be appreciated that other ingredients may be added, for example gylcerol or glucose, to adjust the tonicity of the emulsion. Suitable emulsions will typically contain up to 20% oil, for example, between 5 and 20%. The fat emulsion can comprise fat droplets between 0.1 and 1.0 µm, particularly 0.1 and 0.5 µm, and have a pH in the range of 5.5 to 8.0.

In some embodiments, emulsion compositions are those prepared by mixing a nerve growth factor antagonist with Intralipid™ or the components thereof (soybean oil, egg phospholipids, glycerol and water).

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. The compositions are administered by the oral or nasal respiratory route for local or systemic effect. Compositions in preferably sterile pharmaceutically acceptable solvents may be nebulised by use of gases. Nebulised solutions may be breathed directly from the nebulising device or the nebulising device may be attached to a face mask, tent or intermittent positive pressure breathing machine. Solution, suspension or powder compositions may be administered (including orally or nasally) from devices which deliver the formulation in an appropriate manner.

Treatment efficacy can be assessed by methods well-known in the art.

The following Examples are provided to illustrate but not limit the invention.

### EXAMPLES

### Example 1

### Treatment with anti-NGF monoclonal antibody in conjunction with an opioid analgesic for treating post-surgical pain

We used a pain model that mimics post surgical pain to assess the efficacy of an anti-NGF antibody in conjunction with the opioid analgesic, morphine. Each experiment involved 16 animals (n=8 per group).

Animals. For each experiment, 16 male adult Sprague Dawley rats weighing between 200-220g (Harlan; Indianapolis, IN) were housed under normal light conditions for at least one week prior to use with food and water ad libitum. After a 2 hour period of acclimation in the test chambers the day before surgery, the rats were divided into two groups: one received antibody 15 hours before surgery, the other received vehicle (5% Dextrose/ 0.45% Saline USP) at this time. Anti-NGF antagonist antibody Mab 911 (*see* Hongo, et al., Hybridoma 19:215-227 (2000)) was given at concentrations ranging from 0.3 to 20 mg/ kg intra peritoneal (i.p.). Morphine sulfate was given at various concentrations ranging from 0.1, 0.3, 1 and 3 mg/kg sub-cutaneously (s.c.) 24 hours after surgery to all animals. No animal was given more than two different morphine doses, the doses were at least two hours apart, and the higher dose was always given second. For example, in a typical experiment, half of the animals were given a dose of anti-NGF antibody 15 hours before surgery, intra peritoneally (i.p.), the animals had surgery performed on one hindpaw and were left to recover. 22 hours after surgery, the rats were tested for their "baseline" resting pain and two hours later given the lower dose of morphine (usually 0.3 mg/kg). Thirty minutes after the morphine doses, resting pain was determined as before, and two hours after the initial morphine dose the higher dose was given (usually 1.0 mg/kg). After an additional thirty minutes, resting pain was again determined as below.

The surgery was based on the procedure described by Brennan, et al., Pain 64:493-501 (1996). Animals were anesthetized with a 2% isoflurane and air mixture that was maintained during surgery via a nose cone. The plantar surface of the right hind paw was prepared with a povidone-iodine pad, and a 1-cm central longitudinal incision was made through skin and fascia, starting 0.5 cm from the edge of the heel and extending toward the toes. Measurements were made with a ruler with the foot held in a flexed position. The plantaris muscle was elevated using curved forceps and incised longitudinally. The muscle was incised through its full depth, between the origin and insertion. Bleeding was controlled throughout surgery by pressure applied through a gauze pad. The wound was closed with two mattress sutures (5-0 ethilon black monofilament). These sutures were knotted 5-6 times, with the first knot loosely tied. The wound site was swabbed with bacitracin solution. Animals were allowed to recover and rest in clean cages for at least two hours before behavioral testing began.

Evaluating resting pain. A cumulative pain score was used to assess pain related to weight bearing. Animals were placed on a plastic mesh (grid: 8mm²) in clear plastic cages that were elevated on a platform (h: 18") allowing inspection of the underside of their paws. After a 20 minute acclimation period, weight bearing was assessed on a scale of 0 to 2. A score of 0 was given if the paw was blanched or pressed against the mesh, indicting full weight bearing. A score of 1 was given if the paw was favored with the skin just touching the mesh, with no blanching or indentation of the skin. A score of 2 was given if the paw was held completely off the mesh. Flinching the paw was considered a 2 if the rat was still at rest. Each animal was observed for 1 minute every 5 minutes for 30 minutes. The sum of 6 scores (0-12) obtained during a 1/2-hour period was used to assess pain in the incised foot. Frequency of scores of 2 was also calculated and used to assess the incidence of severe pain or total guarding of the paw by the animal. Each animal was tested 24 hours before surgery (baseline), and 2h, and 24h after surgery. Weight bearing was a good correlate of how willing the animal was to use the limb, and therefore was an effective measure of pain relief.

The following protocol was used for the experiments depicted in Figures 1, 2, and 3. The animals were divided into two groups (control and antibody-treated). Mouse anti-NGF antibody Mab 911 (Hongo et al., supra) was administered i.p. 15 hours before surgery (at 0.3 mg/kg body weight and/or 1 mg/kg body weight, depending on the experiment). Control animals received no antibody but received either no, 0.3 mg/kg, or 1 mg/kg morphine as described herein. Surgery was performed as described above, and resting pain was assessed 22 hours after surgery in both groups ("baseline"). At 24 hours post-surgery, all animals were then treated with morphine at 0.3 mg/kg and resting pain assessed beginning 30 minutes after morphine treatment. A further 1.0 mg/kg of morphine was delivered at 26 hours post surgery and resting pain again assessed starting thirty minutes after this last morphine dose. All morphine treatments were administered subcutaneously, under the scruff of the animal. The results of these experiments are depicted in Figures 1, 2, and 3.

Figure 1 depicts the resting pain score measured in animals that received 0.3 mg/kg of anti-NGF antibody 911 and 0, 0.3 mg/kg body weight or 1.0 mg/kg body weight of morphine. This experiment demonstrated that the cumulative pain score was reduced in animals treated with morphine in combination with an NGF antagonist, anti-NGF antibody 911, compared with treatment with morphine alone or anti-NGF antibody alone. Thus, treatment with anti-NGF antibody in combination with treatment with morphine was more effective in reducing resting pain than morphine alone or anti-NGF antibody alone. These results also showed that treatment with NGF antagonist alone before surgery at 0.3 mg/kg was more effective in reducing resting pain than treatment with 0.3 mg/kg morphine alone.

Figure 2 depicts the resting pain score measured in animals receiving 1.0 mg/kg of anti-NGF antibody 911, and 0, 0.3 mg/kg body weight, or 1 mg/kg body weight of morphine. These results demonstrated that the cumulative pain score was reduced in animals treated with morphine in combination with an anti-NGF antibody, as compared with treatment with anti-NGF antibody alone or morphine alone. Thus, anti-NGF antibody plus morphine was more effective in reducing resting pain than morphine alone or anti-NGF antibody alone. These results also showed that treatment with anti-NGF antibody alone before surgery at 1 mg/kg was more effective in reducing resting pain than treatment with 0.3 mg/kg morphine alone.

Figure 3 depicts the resting pain scores after treatment with 0.3 mg/kg or 1 mg/kg of anti-NGF antibody and with 0, 0.3 mg/kg and 1 mg/kg body weight morphine according to the procedure described above. Treatment with 0.3 mg/kg of morphine in combination with either 0.3 mg/kg anti-NGF antibody or 1 mg/kg anti-NGF antibody significantly improved pain relief as compared with treatment with 0.3 mg/kg morphine alone. The results shown in Figure 3 also demonstrated that treatment with 0.3 mg/kg or 1 mg/kg anti-NGF antibody alone (i.e., in the absence of morphine treatment) provided pain relief equivalent to pain relief following treatment with 0.3 mg/kg dose of morphine. Further, treatment with anti-NGF antibody at 1.0 mg/kg in combination with treatment with morphine at 0.3 mg/kg yielded pain relief at least equal to that obtained with 1 mg/kg of morphine alone. These results demonstrated that treatment with an anti-NGF antibody reduced the amount of morphine needed for effective pain relief.

These experiments demonstrate that treatment with anti-NGF antibody plus morphine is more effective in reducing resting pain than morphine alone or treatment with antibody alone.

## Claims

1. A nerve growth factor (NGF) antagonist and an opioid analgesic for use in treating post-surgical pain whereby the NGF antagonist and the opioid analgesic in conjunction provide effective pain relief; and wherein the NGF antagonist is an anti-NGF antibody which inhibits the binding of NGF to its trkA and p75 receptor *in vivo.*

2. The NGF antagonist and opioid analgesic for use of claim 1, wherein the opioid analgesic is selected from the group consisting of morphine, codeine, dihydrocodeine, diacetylmorphine, hydrocodone, hydromorphone, levorphanol, oxymorphone, alfentanil, buprenorphine, butorphanol, fentanyl, sufentanyl, meperidine, methadone, nalbuphine, propoxyphene and pentazocine.

3. The NGF antagonist and opioid analgesic for use of claim 2, wherein the opioid analgesic is morphine.

4. The NGF antagonist and opioid analgesic for use of any one of claims 1 to 3, wherein the anti-NGF antibody binds human NGF.

5. The NGF antagonist and opioid analgesic for use of claim 4, wherein the anti-NGF antibody binds human NGF with a binding affinity of about 10 nM or less than about 10 nM.

6. The NGF antagonist and opioid analgesic for use of claims 4 or 5, wherein the anti-NGF antibody is a human antibody.

7. The NGF antagonist and opioid analgesic for use of claims 4 or 5, wherein the anti-NGF antibody is a humanized antibody.

8. The NGF antagonist and opioid analgesic for use of any one of the preceding claims, wherein the anti-NGF antibody binds the same human NGF epitope as an antibody comprising the amino acid sequences of SEQ ID NO: 1 and SEQ ID NO: 2.

9. The NGF antagonist and opioid analgesic for use of any one of the preceding claims, wherein the anti-NGF antibody comprises three CDRs from a heavy chain variable region of SEQ ID NO: 1; and three CDRs from a light chain variable region of SEQ ID NO: 2.

10. The NGF antagonist and opioid analgesic for use of any one of the preceding claims, wherein the anti-NGF antibody comprises a heavy chain variable region comprising the sequence of SEQ ID NO: 1 and a light chain variable region comprising the sequence of SEQ ID NO: 2.

11. Use of an NGF antagonist and an opioid analgesic in the manufacture of medicaments for use in combination in a method for treating post-surgical pain whereby the NGF antagonist and the opioid analgesic in conjunction provide effective pain relief; and wherein the NGF antagonist is an anti-NGF antibody which inhibits the binding of NGF to its trkA and p75 receptor *in vivo.*

12. A product comprising an NGF antagonist and an opioid analgesic as a combined preparation for use in treating post-surgical pain wherein the NGF antagonist is an anti-NGF antibody which inhibits the binding of NGF to its trkA and p75 receptor *in vivo.*

13. A pharmaceutical composition comprising an NGF antagonist and an opioid analgesic for use in treating post-surgical pain, wherein the NGF antagonist is an anti-NGF antibody which inhibits the binding of NGF to its trkA and p75 receptor *in vivo.*

14. A kit for use in treating post-surgical pain comprising an NGF antagonist, wherein the NGF antagonist is administered simultaneously, separately or sequentially with an opioid analgesic for the effective treatment of post-surgical pain; wherein the NGF antagonist is an anti-NGF antibody which inhibits the binding of NGF to its trkA and p75 receptor *in vivo.*

## Patentansprüche

1. Nervenwachstumsfaktor(NGF)-Antagonist und Opioidanalgetikum zur Verwendung beim Behandeln von postoperativem Schmerz, wobei der NGF-Antagonist und das Opioidanalgetikum in Verbindung eine wirksame Schmerzlinderung bereitstellen; und wobei der NGF-Antagonist ein Anti-NGF-Antikörper ist, der die Bindung von NGF an seinen trkA- und p75-Rezeptor *in vivo* inhibiert.

2. NGF-Antagonist und Opioidanalgetikum zur Verwendung gemäß Anspruch 1, wobei das Opioidanalgetikum ausgewählt ist aus der Gruppe, bestehend aus Morphin, Codein, Dihydrocodein, Diacetylmorphin, Hydrocodon, Hydromorphon, Levorphanol, Oxymorphon, Alfentanil, Buprenorphin, Butorphanol, Fentanyl, Sufentanyl, Meperidin, Methadon, Nalbuphin, Propoxyphen und Pentazocin.

3. NGF-Antagonist und Opioidanalgetikum zur Verwendung gemäß Anspruch 2, wobei das Opioidanalgetikum Morphin ist.

4. NGF-Antagonist und Opioidanalgetikum zur Verwendung gemäß einem der Ansprüche 1 bis 3, wobei der Anti-NGF-Antikörper humanen NGF bindet.

5. NGF-Antagonist und Opioidanalgetikum zur Verwendung gemäß Anspruch 4, wobei der Anti-NGF-Antikörper humanen NGF mit einer Bindungsaffinität von etwa 10 nM oder weniger als etwa 10 nM bindet.

6. NGF-Antagonist und Opioidanalgetikum zur Verwendung gemäß Anspruch 4 oder 5, wobei der Anti-NGF-Antikörper ein Humanantikörper ist.

7. NGF-Antagonist und Opioidanalgetikum zur Verwendung gemäß Anspruch 4 oder 5, wobei der Anti-NGF-Antikörper ein humanisierter Antikörper ist.

8. NGF-Antagonist und Opioidanalgetikum zur Verwendung gemäß einem der vorhergehenden Ansprüche, wobei der Anti-NGF-Antikörper dasselbe humane NGF-Epitop bindet wie ein Antikörper, der die Aminosäuresequenzen SEQ ID NO:1 und SEQ ID NO:2 umfasst.

9. NGF-Antagonist und Opioidanalgetikum zur Verwendung gemäß einem der vorhergehenden Ansprüche, wobei der Anti-NGF-Antikörper drei CDR aus der variablen Region der schweren Kette von SEQ ID NO:1 und drei CDR aus einer variablen Region der leichten Kette von SEQ ID NO:2 umfasst.

10. NGF-Antagonist und Opioidanalgetikum zur Verwendung gemäß einem der vorhergehenden Ansprüche, wobei der Anti-NGF-Antikörper eine variable Region der schweren Kette, umfassend die Sequenz SEQ ID NO:1, und eine variable Region der leichten Kette, umfassend die Sequenz SEQ ID NO:2, umfasst.

11. Verwendung eines NGF-Antagonisten und eines Opioidanalgetikums bei der Herstellung von Medikamenten zur Verwendung in Kombination in einem Verfahren zum Behandeln von postoperativem Schmerz, wobei der NGF-Antagonist und das Opioidanalgetikum in Verbindung eine wirksame Schmerzlinderung bereitstellen; und wobei der NGF-Antagonist ein Anti-NGF-Antikörper ist, der die Bindung von NGF an seinen trkA- und p75-Rezeptor *in vivo* inhibiert.

12. Produkt, umfassend einen NGF-Antagonisten und ein Opioidanalgetikum als Kombinationspräparat zur Verwendung beim Behandeln von postoperativem Schmerz, wobei der NGF-Antagonist ein Anti-NGF-Antikörper ist, der die Bindung von NGF an seinen trkA- und p75-Rezeptor *in vivo* inhibiert.

13. Pharmazeutische Zusammensetzung, umfassend einen NGF-Antagonisten und ein Opioid-Analgetikum zur Verwendung beim Behandeln von postoperativem Schmerz, wobei der NGF-Antagonist ein Anti-NGF-Antikörper ist, der die Bindung von NGF an seinen trkA- und p75-Rezeptor *in vivo* inhibiert.

14. Kit zur Verwendung beim Behandeln von postoperativem Schmerz, umfassend einen NGF-Antagonisten, wobei der NGF-Antagonist gleichzeitig, getrennt oder sequenziell mit einem Opioidanalgetikum für die wirksame Behandlung von postoperativem Schmerz verabreicht wird; wobei der NGF-Antagonist ein Anti-NGF-Antikörper ist, der die Bindung von NGF an seinen trkA- und p75-Rezeptor *in vivo* inhibiert.

## Revendications

1. Antagoniste du facteur de croissance des nerfs (NGF) et analgésique opioïde pour leur utilisation dans le traitement de la douleur post-chirurgicale, moyennant quoi l'antagoniste du NGF et l'analgésique opioïde apportent conjointement un soulagement efficace de la douleur ; et dans lesquels l'antagoniste du NGF est un anticorps anti-NGF qui inhibe la liaison du NGF à ses récepteurs trkA et p75 *in vivo.*

2. Antagoniste du NGF et analgésique opioïde pour leur utilisation selon la revendication 1, dans lesquels l'analgésique opioïde est sélectionné dans le groupe consistant en la morphine, la codéine, la dihydrocodéine, la diacétylmorphine, l'hydrocodone, l'hydromorphone, le lévorphanol, l'oxymorphone, l'alfentanil, la buprénorphine, le butorphanol, le fentanyl, le sufentanyl, la mépéridine, la méthadone, la nalbuphine, le propoxyphène et la pentazocine.

3. Antagoniste du NGF et analgésique opioïde pour leur utilisation selon la revendication 2, dans lesquels l'analgésique opioïde est la morphine.

4. Antagoniste du NGF et analgésique opioïde pour leur utilisation selon l'une des revendications 1 à 3, dans lesquels l'anticorps anti-NGF se lie au NGF humain.

5. Antagoniste du NGF et analgésique opioïde pour leur utilisation selon la revendication 4, dans lesquels l'anticorps anti-NGF se lie au NGF humain avec une affinité de liaison d'environ 10 nM ou inférieure à environ 10 nM.

6. Antagoniste du NGF et analgésique opioïde pour leur utilisation selon les revendications 4 ou 5, dans lesquels l'anticorps anti-NGF est un anticorps humain.

7. Antagoniste du NGF et analgésique opioïde pour leur utilisation selon les revendications 4 ou 5, dans lesquels l'anticorps anti-NGF est un anticorps humanisé.

8. Antagoniste du NGF et analgésique opioïde pour leur utilisation selon l'une des revendications précédentes, dans lesquels l'anticorps anti-NGF se lie au même épitope du NGF humain qu'un anticorps comprenant les séquences d'acides aminés de SEQ ID NO : 1 et SEQ ID NO : 2.

9. Antagoniste du NGF et analgésique opioïde pour leur utilisation selon l'une des revendications précédentes, dans lesquels l'anticorps anti-NGF comprend trois CDR d'une région variable de chaîne lourde de SEQ ID NO : 1, et trois CDR d'une région variable de chaîne légère de SEQ ID NO : 2.

10. Antagoniste du NGF et analgésique opioïde pour leur utilisation selon l'une des revendications précédentes, dans lesquels l'anticorps anti-NGF comprend une région variable de chaîne lourde comprenant la séquence de SEQ ID NO : 1, et une région variable de chaîne légère comprenant la séquence de SEQ ID NO : 2.

11. Utilisation d'un antagoniste du NGF et d'un analgésique opioïde dans la fabrication de médicaments pour leur utilisation en combinaison dans un procédé de traitement de la douleur post-chirurgicale, moyennant quoi l'antagoniste du NGF et l'analgésique opioïde apportent conjointement un soulagement efficace de la douleur ; et dans laquelle l'antagoniste du NGF est un anticorps anti-NGF qui inhibe la liaison du NGF à ses récepteurs trkA et p75 *in vivo.*

12. Produit comprenant un antagoniste du NGF et un analgésique opioïde sous la forme d'une préparation combinée pour leur utilisation dans le traitement de la douleur post-chirurgicale, dans lequel l'antagoniste du NGF est un anticorps anti-NGF qui inhibe la liaison du NGF à ses récepteurs trkA et p75 *in vivo.*

13. Composition pharmaceutique comprenant un antagoniste du NGF et un analgésique opioïde pour son utilisation dans le traitement de la douleur post-chirurgicale, dans laquelle l'antagoniste du NGF est un anticorps anti-NGF qui inhibe la liaison du NGF à ses récepteurs trkA et p75 *in vivo.*

14. Kit pour son utilisation dans le traitement de la douleur post-chirurgicale comprenant un antagoniste du NGF, dans lequel l'antagoniste du NGF et l'analgésique opioïde sont administrés simultanément, séparément ou successivement pour un traitement efficace de la douleur post-chirurgicale ; dans lequel l'antagoniste du NGF est un anticorps anti-NGF qui inhibe la liaison du NGF à ses récepteurs trkA et p75 *in vivo.*
